# EUROPEAN PATENT APPLICATION

(11) **EP 3 575 409 A1**
(43) Date of publication of application: **04.12.2019**
(21) Application number: 18744850.1
(22) Date of filing: 26.01.2018
(51) Int. Cl.: C12Q 1/68, G01N 33/53

(54) **METHOD FOR ASSESSING ANATOMICAL SITE OF BODY SURFACE TISSUE**

(30) Priority: 27.01.2017 JP 2017013414
(71) Applicant: Osaka University, Suita-shi, Osaka 565-0871 (JP)
(72) Inventor: NISHIDA, Kohji, Suita-shi Osaka 565-0871 (JP); HAYASHI, Ryuhei, Suita-shi Osaka 565-0871 (JP); OKUBO, Toru, Suita-shi Osaka 565-0871 (JP)
(74) Representative: Witte, Weller & Partner Patentanwälte mbB
(86) International application number: PCT/JP2018/002594
(87) International publication number: WO 2018/139618

(57) **Abstract**

An object of the present invention is to provide a method for determining anatomical sites of a body surface tissue. Provided is a method for determining an anatomical site of origin of a body surface tissue specimen, the method comprising the steps of:
(A) selecting at least 8 kinds of genes from the group consisting of a Hox gene family and a Pax6 gene;
(B) measuring the expression levels of the genes selected in the above (A) in the body surface tissue specimen; and
(C) determining the anatomical site of origin of the body surface tissue specimen based on the expression levels or a combination of the expression levels measured in the above (B) .

## Description

### TECHNICAL FIELD

The present invention relates to a method for determining anatomical sites of a body surface tissue.

### BACKGROUND ART

The skin continuously covers the entire outer surface of the body, but its appearance and function vary with the site. For example, the skin in the upper arms and the abdomen is more elastic than that in the palm (Non Patent Literature 1). Also, the skin in the precordia, the shoulders, the earlobes, the upper arms and the cheeks is more susceptible to keloid formation than that in the other sites (Non Patent Literature 2). In addition, transplantation of trunk epidermis into a damaged palm or sole of a patient results in a higher incident of ulcers (Non Patent Literature 3). As just described, the skin is known to vary in nature with the site.

In the course of development, the body cells of living organisms acquire positional information from homeobox genes and differentiate appropriately in particular sites. The homeobox genes, which were first discovered in drosophila, are present in vertebrates as well, and the DNA sequences of the homeobox genes are conserved across different species of living organisms (Non Patent Literature 4). Therefore, the homeobox genes are considered as a one of the most important gene families for the ontogenesis and morphogenesis of living organisms. According to recent reports, even in adults, positional information is retained in fibroblasts by the expression of several homeobox genes (Non Patent Literature 5 and Non Patent Literature 6). However, it is not understood in detail how homeobox genes function to provide positional information in the other organs in adults.

### CITATION LIST

### Non Patent Literature

Non Patent Literature 1: Arch Dermatol Res. 282, 283-288, 1990 Non Patent Literature 2: Mol Med. 17, 113-125, 2011
Non Patent Literature 3: J. Dermatol. Sci. 40, 1-9, 2005 Non Patent Literature 4: Science. 249, 374-379, 1990
Non Patent Literature 5: Genes Dev., 22, 303-307, 2008
Non Patent Literature 6: J. Invest. Dermatol., 128, 776-782, 2008

### SUMMARY OF INVENTION

### TECHNICAL PROBLEM

An object of the present invention is to provide a method for determining anatomical sites of a body surface tissue.

### SOLUTION TO PROBLEM

The present inventors conducted extensive research to achieve the above-mentioned object. The present inventors measured the expression levels of homeobox genes in 24 specimens excised from different sites of a mouse body surface tissue, and as a result, found that the expression levels and patterns vary with the anatomical site of the body surface tissue. Through further examination, the present inventors obtained new findings, and then completed the present invention.

That is, the present invention relates to the following.
[1] A method for determining an anatomical site of origin of a body surface tissue specimen, the method comprising the steps of:
   (A) selecting at least one gene of a homeobox gene family;
   (B) measuring the expression level of the at least one gene selected in the above (A) in the body surface tissue specimen; and
   (C) determining the anatomical site of origin of the body surface tissue specimen based on the expression level or a combination of the expression levels measured in the above (B) .
[2] The method according to the above [1], wherein the homeobox gene family consists of a Hox gene family and a Pax6 gene.
[3] The method according to the above [2], wherein the HOX gene family consists of Hoxa1 to Hoxa4, Hoxa6, Hoxa7, Hoxa9 to Hoxa11, and Hoxa13; Hoxb3 to Hoxb9, and Hoxb13; Hoxc4 to Hoxc6, Hoxc8, and Hoxc10 to Hoxc13; and Hoxd4, and Hoxd8 to Hoxd13.
[4] The method according to any one of the above [1] to [3], wherein the anatomical site of origin of the body surface tissue specimen is at least one site selected from cornea-right, cornea-left, eyelid-right, eyelid-left, ear-right, ear-left, cheek-right, cheek-left, top, neck-ventral, neck-dorsal, trunk-upper ventral, trunk-upper dorsal, trunk-lower ventral, trunk-lower dorsal, foreleg-right, foreleg-left, forefoot-right, forefoot-left, hindleg-right, hindleg-left, hindfoot-right, hindfoot-left, and tail.
[5] The method according to any one of the above [1] to [4], wherein, in step (A), the genes specified in Table 6, namely, the following 8 kinds of genes:
   1: Hoxa3 or Hoxa6,
   2: Hoxb4,
   3: Hoxb5,
   4: Hoxb8,
   5: Hoxb9,
   6: Hoxc10, Hoxc11, or Hoxc12,
   7: Hoxd4, and
   8: Pax6
   are selected, and
   wherein, in step (C), the expression of each selected gene is determined to be positive or negative, and the anatomical site of origin of the body surface tissue specimen is determined with reference to the gene expression patterns specified in Table 6.
[6] The method according to any one of the above [1] to [4], wherein, in step (A), the genes specified in Table 9, namely, the following 10 kinds of genes:
   1: Hoxa3 or Hoxa6,
   2: Hoxa4,
   3: Hoxb4,
   4: Hoxb5, Hoxb6, or Hoxb7,
   5: Hoxb8,
   6: Hoxb9,
   7: Hoxc10, Hoxc11, or Hoxc12,
   8: Hoxd4,
   9: Hoxd12, and
   10: Pax6
   are selected, and
   wherein, in step (C), the expression of each selected gene is determined to be positive or negative, and the anatomical site of origin of the body surface tissue specimen is determined with reference to the gene expression patterns specified in Table 9.
[7] The method according to the above [5] or [6], wherein a first anatomical site determination comprises determining the anatomical site of origin of the body surface tissue specimen with reference to the expression pattern of at least one gene selected from the group consisting of Hoxa3 and Hoxa6.
[8] The method according to any one of the above [1] to [4], wherein, in step (A), the genes specified in Table 12, namely, the following 11 kinds of genes:
   1: Hoxa3 or Hoxa6,
   2: Hoxa9,
   3: Hoxa13, Hoxc10, Hoxc11, or Hoxc12,
   4: Hoxb3 or Hoxb7,
   5: Hoxb4,
   6: Hoxb9,
   7: Hoxb13, Hoxd9, or Hoxd10,
   8: Hoxc4,
   9: Hoxd4,
   10: Hoxd11, and
   11: Pax6
   are selected, and
   wherein, in step (C), the expression of each selected gene is determined to be positive or negative, and the anatomical site of origin of the body surface tissue specimen is determined with reference to the gene expression patterns specified in Table 12.
[9] The method according to the above [8], wherein a first anatomical site determination comprises determining the anatomical site of origin of the body surface tissue specimen with reference to the expression pattern of at least one gene selected from the group consisting of Hoxb13, Hoxd9 and Hoxd10.
[10] The method according to any one of the above [1] to [4], wherein, in step (A), one gene selected from the group consisting of the Hoxa3 gene, the Hoxa6 gene, the Hoxb3 gene, the Hoxb4 gene, the Hoxb5 gene, the Hoxb6 gene and the Hoxb7 gene is selected, and
   wherein, in step (C), the expression of the selected gene is determined to be positive or negative; and in the case of positive expression of the selected gene, the anatomical site of origin of the body surface tissue specimen is determined to be a posterior region, and in the case of negative expression of the selected gene, the anatomical site of origin of the body surface tissue specimen is determined to be an anterior region.
[11] The method according to any one of the above [1] to [4], wherein, in step (A), one gene selected from the group consisting of the Hoxa9 gene, the Hoxa11 gene, the Hoxa13 gene, the Hoxb13 gene, the Hoxc10 gene, the Hoxc11 gene, the Hoxc12 gene, the Hoxd9 gene, the Hoxd10 gene, and the Hoxd13 gene is selected, and
   wherein, in step (C), the expression of the selected gene is determined to be positive or negative; and in the case of positive expression of the selected gene, the anatomical site of origin of the body surface tissue specimen is determined to be a distal region, and in the case of negative expression of the selected gene, the anatomical site of origin of the body surface tissue specimen is determined to be a proximal region.
[12] The method according to any one of the above [1] to [4], wherein, in step (A), the Hoxb9 gene or the Hoxd4 gene is selected, and
   wherein, in step (C), the expression of the selected gene is determined to be positive or negative; and in the case of positive expression of the selected gene, the anatomical site of origin of the body surface tissue specimen is determined to be a dorsal region, and in the case of negative expression of the selected gene, the anatomical site of origin of the body surface tissue specimen is determined to be a ventral region.
[13] The method according to the above [4], wherein the anatomical site of origin of the body surface tissue specimen is determined in any of the following manners:
   (1) in step (A), the Hoxa3 gene or the Hoxa6 gene is selected, and
      in step (C), the expression of the selected gene is determined to be positive or negative; and in the case of positive expression of the selected gene, the anatomical site of origin of the body surface tissue specimen is determined to be trunk, hindleg, hindfoot or tail as set forth in the above [4], and in the case of negative expression of the selected gene, the anatomical site of origin of the body surface tissue specimen is determined to be any of the other anatomical sites as set forth in the above [4];
   (2) in step (A), the Hoxb3 gene or the Hoxb7 gene is selected, and
      in step (C), the expression of the selected gene is determined to be positive or negative; and in the case of positive expression of the selected gene, the anatomical site of origin of the body surface tissue specimen is determined to be neck-dorsal, trunk, hindleg, hindfoot or tail as set forth in the above [4], and in the case of negative expression of the selected gene, the anatomical site of origin of the body surface tissue specimen is determined to be any of the other anatomical sites as set forth in the above [4];
   (3) in step (A), the Hoxb4 gene is selected, and
      in step (C), the expression of the selected gene is determined to be positive or negative; and in the case of positive expression of the selected gene, the anatomical site of origin of the body surface tissue specimen is determined to be neck, trunk, foreleg, hindleg, hindfoot or tail as set forth in the above [4], and in the case of negative expression of the selected gene, the anatomical site of origin of the body surface tissue specimen is determined to be any of the other anatomical sites as set forth in the above [4];
   (4) in step (A), the Hoxb5 gene or the Hoxb6 gene is selected, and
      in step (C), the expression of the selected gene is determined to be positive or negative; and in the case of positive expression of the selected gene, the anatomical site of origin of the body surface tissue specimen is determined to be neck-dorsal, trunk, hindleg, hindfoot-right or tail as set forth in the above [4], and in the case of negative expression of the selected gene, the anatomical site of origin of the body surface tissue specimen is determined to be any of the other anatomical sites as set forth in the above [4];
   (5) in step (A), the Hoxa9 gene is selected, and in step (C), the expression of the selected gene is determined to be positive or negative; and in the case of positive expression of the selected gene, the anatomical site of origin of the body surface tissue specimen is determined to be trunk-lower, forefoot-right, hindleg, hindfoot or tail as set forth in the above [4], and in the case of negative expression of the selected gene, the anatomical site of origin of the body surface tissue specimen is determined to be any of the other anatomical sites as set forth in the above [4];
   (6) in step (A), the Hoxa11 gene is selected, and in step (C), the expression of the selected gene is determined to be positive or negative; and in the case of positive expression of the selected gene, the anatomical site of origin of the body surface tissue specimen is determined to be forefoot-right or tail as set forth in the above [4], and in the case of negative expression of the selected gene, the anatomical site of origin of the body surface tissue specimen is determined to be any of the other anatomical sites as set forth in the above [4];
   (7) in step (A), the Hoxa13 gene, the Hoxc10 gene, the Hoxc11 gene, or the Hoxc12 gene is selected, and
      in step (C), the expression of the selected gene is determined to be positive or negative; and in the case of positive expression of the selected gene, the anatomical site of origin of the body surface tissue specimen is determined to be forefoot, hindfoot or tail as set forth in the above [4], and in the case of negative expression of the selected gene, the anatomical site of origin of the body surface tissue specimen is determined to be any of the other anatomical sites as set forth in the above [4] ;
   (8) in step (A), the Hoxb13 gene, the Hoxd9 gene, or the Hoxd10 gene is selected, and
      in step (C), the expression of the selected gene is determined to be positive or negative; and in the case of positive expression of the selected gene, the anatomical site of origin of the body surface tissue specimen is determined to be tail as set forth in the above [4], and in the case of negative expression of the selected gene, the anatomical site of origin of the body surface tissue specimen is determined to be any of the other anatomical sites as set forth in the above [4];
   (9) in step (A), the Hoxd13 gene is selected, and
      in step (C), the expression of the selected gene is determined to be positive or negative; and in the case of positive expression of the selected gene, the anatomical site of origin of the body surface tissue specimen is determined to be tail as set forth in the above [4], and in the case of negative expression of the selected gene, the anatomical site of origin of the body surface tissue specimen is determined to be any of the other anatomical sites as set forth in the above [4];
   (10) in step (A), the Hoxb9 gene is selected, and
      in step (C), the expression of the selected gene is determined to be positive or negative; and in the case of positive expression of the selected gene, the anatomical site of origin of the body surface tissue specimen is determined to be trunk-upper dorsal, trunk-lower dorsal, hindleg or tail as set forth in the above [4], and in the case of negative expression of the selected gene, the anatomical site of origin of the body surface tissue specimen is determined to be any of the other anatomical sites as set forth in the above [4];
   (11) in step (A), the Hoxd4 gene is selected, and
      in step (C), the expression of the selected gene is determined to be positive or negative; and in the case of positive expression of the selected gene, the anatomical site of origin of the body surface tissue specimen is determined to be trunk-upper dorsal, trunk-lower dorsal or tail as set forth in the above [4], and in the case of negative expression of the selected gene, the anatomical site of origin of the body surface tissue specimen is determined to be any of the other anatomical sites as set forth in the above [4];
   (12) in step (A), the Hoxa1 gene is selected, and
      in step (C), the expression of the selected gene is determined to be positive or negative; and in the case of negative expression of the selected gene, the anatomical site of origin of the body surface tissue specimen is determined to be cornea as set forth in the above [4], and in the case of positive expression of the selected gene, the anatomical site of origin of the body surface tissue specimen is determined to be any of the other anatomical sites as set forth in the above [4];
   (13) in step (A), the Hoxa2 gene is selected, and
      in step (C), the expression of the selected gene is determined to be positive or negative; and in the case of negative expression of the selected gene, the anatomical site of origin of the body surface tissue specimen is determined to be cornea, forefoot, hindfoot or tail as set forth in the above [4], and in the case of positive expression of the selected gene, the anatomical site of origin of the body surface tissue specimen is determined to be any of the other anatomical sites as set forth in the above [4];
   (14) in step (A), the Hoxa4 gene is selected, and
      in step (C), the expression of the selected gene is determined to be positive or negative; and in the case of negative expression of the selected gene, the anatomical site of origin of the body surface tissue specimen is determined to be cornea, ear-right, cheek-left, hindfoot-right or tail as set forth in the above [4], and in the case of positive expression of the selected gene, the anatomical site of origin of the body surface tissue specimen is determined to be any of the other anatomical sites as set forth in the above [4];
   (15) in step (A), the Hoxa7 gene is selected, and
      in step (C), the expression of the selected gene is determined to be positive or negative; and in the case of negative expression of the selected gene, the anatomical site of origin of the body surface tissue specimen is determined to be cornea, ear-right or foreleg-right as set forth in the above [4], and in the case of positive expression of the selected gene, the anatomical site of origin of the body surface tissue specimen is determined to be any of the other anatomical sites as set forth in the above [4];
   (16) in step (A), the Hoxa10 gene is selected, and
      in step (C), the expression of the selected gene is determined to be positive or negative; and in the case of negative expression of the selected gene, the anatomical site of origin of the body surface tissue specimen is determined to be cornea, ear-right, neck-dorsal, trunk-upper or trunk-lower dorsal as set forth in the above [4], and in the case of positive expression of the selected gene, the anatomical site of origin of the body surface tissue specimen is determined to be any of the other anatomical sites as set forth in the above [4];
   (17) in step (A), the Hoxb8 gene is selected, and
      in step (C), the expression of the selected gene is determined to be positive or negative; and in the case of negative expression of the selected gene, the anatomical site of origin of the body surface tissue specimen is determined to be cornea or ear-right as set forth in the above [4], and in the case of positive expression of the selected gene, the anatomical site of origin of the body surface tissue specimen is determined to be any of the other anatomical sites as set forth in the above [4] ;
   (18) in step (A), the Hoxc4 gene is selected, and
      in step (C), the expression of the selected gene is determined to be positive or negative; and in the case of negative expression of the selected gene, the anatomical site of origin of the body surface tissue specimen is determined to be cornea, eyelid, ear, cheek, forefoot-left, hindfoot or tail as set forth in the above [4], and in the case of positive expression of the selected gene, the anatomical site of origin of the body surface tissue specimen is determined to be any of the other anatomical sites as set forth in the above [4];
   (19) in step (A), the Hoxc5 gene or the Hoxc6 gene is selected, and
      in step (C), the expression of the selected gene is determined to be positive or negative; and in the case of negative expression of the selected gene, the anatomical site of origin of the body surface tissue specimen is determined to be cornea, eyelid, ear, cheek, top or tail as set forth in the above [4], and in the case of positive expression of the selected gene, the anatomical site of origin of the body surface tissue specimen is determined to be any of the other anatomical sites as set forth in the above [4];
   (20) in step (A), the Hoxc8 gene is selected, and
      in step (C), the expression of the selected gene is determined to be positive or negative; and in the case of negative expression of the selected gene, the anatomical site of origin of the body surface tissue specimen is determined to be cornea, eyelid, ear, cheek, top, neck-dorsal, hindfoot or tail as set forth in the above [4], and in the case of positive expression of the selected gene, the anatomical site of origin of the body surface tissue specimen is determined to be any of the other anatomical sites as set forth in the above [4];
   (21) in step (A), the Hoxc13 gene is selected, and
      in step (C), the expression of the selected gene is determined to be positive or negative; and in the case of negative expression of the selected gene, the anatomical site of origin of the body surface tissue specimen is determined to be cornea, ear or trunk-lower ventral as set forth in the above [4], and in the case of positive expression of the selected gene, the anatomical site of origin of the body surface tissue specimen is determined to be any of the other anatomical sites as set forth in the above [4];
   (22) in step (A), the Hoxd8 gene is selected, and
      in step (C), the expression of the selected gene is determined to be positive or negative; and in the case of negative expression of the selected gene, the anatomical site of origin of the body surface tissue specimen is determined to be cornea, ear, forefoot or hindfoot-right as set forth in the above [4], and in the case of positive expression of the selected gene, the anatomical site of origin of the body surface tissue specimen is determined to be any of the other anatomical sites as set forth in the above [4];
   (23) in step (A), the Hoxd11 gene is selected, and
      in step (C), the expression of the selected gene is determined to be positive or negative; and in the case of negative expression of the selected gene, the anatomical site of origin of the body surface tissue specimen is determined to be cornea, ear, neck-ventral, trunk-upper ventral, forefoot-left or hindfoot-right as set forth in the above [4], and in the case of positive expression of the selected gene, the anatomical site of origin of the body surface tissue specimen is determined to be any of the other anatomical sites as set forth in the above [4] ;
   (24) in step (A), the Hoxd12 gene is selected, and
      in step (C), the expression of the selected gene is determined to be positive or negative; and in the case of negative expression of the selected gene, the anatomical site of origin of the body surface tissue specimen is determined to be cornea, ear-right, top, neck-dorsal or trunk-upper dorsal as set forth in the above [4], and in the case of positive expression of the selected gene, the anatomical site of origin of the body surface tissue specimen is determined to be any of the other anatomical sites as set forth in the above [4]; and
   (25) in step (A), the Pax6 gene is selected, and
      in step (C), the expression of the selected gene is determined to be positive or negative; and in the case of positive expression of the selected gene, the anatomical site of origin of the body surface tissue specimen is determined to be cornea or eyelid-right as set forth in the above [4], and in the case of negative expression of the selected gene, the anatomical site of origin of the body surface tissue specimen is determined to be any of the other anatomical sites as set forth in the above [4] .
[14] The method according to any one of the above [1] to [13], wherein the body surface tissue specimen is epidermis or cornea.
[15] The method according to any one of the above [1] to [14], wherein the epidermis or the cornea is prepared by excision or fabrication, or by excision or fabrication followed by culture.

### ADVANTAGEOUS EFFECTS OF INVENTION

The present invention provides a method for determining anatomical sites of a body surface tissue. In the method of the present invention, a combination of specific genes is selected from a homeobox gene family, their expression levels are measured, and based on the measurement results, a large number of anatomical sites can be determined using a small number of genes in a simple manner. In this regard, the method of the present invention is markedly effective.

### BRIEF DESCRIPTION OF DRAWINGS

Fig. 1 shows the anatomically defined sites of the body surface tissue and the name of each site.
Fig. 2A shows the results of the analysis of the expression levels of Hoxa genes in the indicated sites of the body surface tissue.
Fig. 2B shows the results of the analysis of the expression levels of Hoxb genes in the indicated sites of the body surface tissue.
Fig. 2C shows the results of the analysis of the expression levels of Hoxc genes in the indicated sites of the body surface tissue.
Fig. 2D shows the results of the analysis of the expression levels of Hoxd genes, the Pax6 gene and the K14 gene in the indicated sites of the body surface tissue.
Fig. 3 shows gene groups which can be used as indices to distinguish between anatomical regions (anterior and posterior regions, proximal and distal regions, dorsal and ventral regions, and the others) of the body surface tissue.
Fig. 4 shows a flowchart for determination of a maximum of 11 anatomical sites of the body surface tissue using a minimum of 8 kinds of genes.
Fig. 5 shows a flowchart for determination of a maximum of 14 anatomical sites of the body surface tissue using a minimum of 10 kinds of genes.
Fig. 6 shows a flowchart for determination of a maximum of 15 anatomical sites of the body surface tissue using a minimum of 11 kinds of genes.

### DESCRIPTION OF EMBODIMENTS

The present invention provides a method for determining an anatomical site of origin of a body surface tissue specimen, the method comprising the steps of:
(A) selecting at least one gene of a homeobox gene family;
(B) measuring the expression level of the at least one gene selected in the above (A) in the body surface tissue specimen; and
(C) determining the anatomical site of origin of the body surface tissue specimen based on the expression level or a combination of the expression levels measured in the above (B) .

In the present invention, the body surface tissue is an epithelial tissue. The epithelial tissue may be, for example, skin, cornea or the like.

In the present invention, the body surface tissue specimen may be, for example, an excised or fabricated body surface tissue or the like. Alternatively, the body surface tissue specimen may be prepared, for example, by culturing an excised or fabricated body surface tissue or the like.

In the present invention, animals that can be subjects for the determination of anatomical sites of the body surface tissue are, for example, mammals. The mammals include, for example, primates such as humans, monkeys, orangutans, chimpanzees and gorillas; experimental animals including rodents, such as mice, rats, hamsters and guinea pigs, and rabbits; domestic animals such as cattle, horses, pigs, sheep and goats; pet animals such as dogs and cats; and birds such as chickens, domestic ducks and geese. The mammals are preferably rodents (mice etc.) or primates (humans etc.), more preferably mice or humans, and still more preferably mice.

In the present invention, the body surface tissue specimen prepared by excision may be, for example, a body surface tissue excised from the living body or the like by surgery or the like.

In the present invention, the body surface tissue specimen prepared by fabrication may be a body surface tissue experimentally fabricated. For example, a body surface tissue specimen may be prepared by induced differentiation or the like of induced pluripotent stem cells (iPS cells), embryonic stem cells (ES cells), mesenchymal stem cells or the like, or by reprogramming of fibroblasts, followed by induced differentiation or the like.

In the present invention, the body surface tissue specimen prepared by excision, fabrication or the like followed by culture is preferably characterized, for example, in that the expression pattern of HOX genes does not change before and after the culture. In order to perform the culture without changing the expression pattern of HOX genes in the body surface tissue specimen, for example, an excised or fabricated body surface tissue may be cultured in DMEM/F12 culture medium supplemented with a medium supplement (e.g., 2% B27 supplement), a ROCK inhibitor (e.g., 10 µM Y-27632) and a cell growth factor in a known cultureware (e.g., iMatrix-511-coated dish). In the case where the excised or fabricated body surface tissue is a population of epidermal keratinocytes, the culture is preferably performed in DMEM/F12 culture medium (containing 2% B27 supplement and 10 µM Y-27632) supplemented with 10 ng/mL EGF as a cell growth factor. In the case where the excised or fabricated body surface tissue is a population of corneal epithelial cells, the culture is preferably performed in DMEM/F12 culture medium (containing 2% B27 supplement and 10 µM Y-27632) supplemented with 20 ng/mL KGF as a cell growth factor.

In the present invention, the anatomical site is, for example, an anatomically defined site. Examples of the anatomically defined site include cornea-right, cornea-left, eyelid-right, eyelid-left, ear-right, ear-left, cheek-right, cheek-left, top, neck-ventral, neck-dorsal, trunk-upper ventral, trunk-upper dorsal, trunk-lower ventral, trunk-lower dorsal, foreleg-right, foreleg-left, forefoot-right, forefoot-left, hindleg-right, hindleg-left, hindfoot-right, hindfoot-left, and tail. Also included are an anterior region, a posterior region, a proximal region, a distal region, a dorsal region, a ventral region, etc.

In the present invention, the anterior region as an anatomically defined site includes cornea-right, cornea-left, eyelid-right, eyelid-left, ear-right, ear-left, cheek-right, cheek-left, top, neck-ventral, neck-dorsal, foreleg-right, foreleg-left, forefoot-right, and forefoot-left.

In the present invention, the posterior region as an anatomically defined site includes neck-ventral, neck-dorsal, trunk-upper ventral, trunk-upper dorsal, trunk-lower ventral, trunk-lower dorsal, foreleg-right, foreleg-left, hindleg-right, hindleg-left, hindfoot-right, hindfoot-left, and tail.

In the present invention, the proximal region as an anatomically defined site includes cornea-right, cornea-left, eyelid-right, eyelid-left, ear-right, ear-left, cheek-right, cheek-left, top, neck-ventral, neck-dorsal, trunk-upper ventral, trunk-upper dorsal, trunk-lower ventral, trunk-lower dorsal, foreleg-right, foreleg-left, hindleg-right, and hindleg-left.

In the present invention, the distal region as an anatomically defined site includes trunk-lower ventral, trunk-lower dorsal, forefoot-right, forefoot-left, hindleg-right, hindleg-left, hindfoot-right, hindfoot-left, and tail.

In the present invention, the dorsal region as an anatomically defined site includes trunk-upper dorsal, trunk-lower dorsal, hindleg-right, hindleg-left and tail.

In the present invention, the ventral region as an anatomically defined site includes trunk-upper ventral and trunk-lower ventral.

In step (A), at least one gene of the homeobox gene family is selected.

The members of the homeobox gene family are genes each having a homeobox domain in the sequence. The members of the homeobox gene family are, for example, a Hox gene family, a Pax gene family, a DLX gene family, an IRX gene family, an MEIS gene family, an MKX gene, a PBX gene family, a PKNOX gene family, an ADNF gene, an ALX gene family, an ARGFX gene, an ARX gene, a BARHL gene family, a BARX gene family, a CDX gene family, a CRX gene, a CUTL gene family, a DBX gene family, a DMBX gene, a DPRX gene, a DRGX gene, a DUX gene family, an EMX gene family, an EN gene family, an ESX1L gene, an FVX gene family, a GBX gene family, a GSC gene family, a GSX gene family, an HESX1 gene, an HHEX gene, an HLX1 gene, an HMBOX1 gene, an HMX gene family, an HNF gene family, an HOMEZ gene, an HOPX gene, an ISL gene family, an ISX gene, an LASS gene family, an LBX gene family, an LHX gene family, an LMX gene family, an MEOX gene family, an MIXL1 gene, an MNX1 gene, an MSX gene family, an NANOG gene, an NKX gene family, an NOBOX gene, an NOTO gene, an ONECUT gene family, an OTP gene, an OTX gene family, a PDX1 gene, a PHOX gene family, a PITX gene family, a PROP1 gene, a PRRX gene family, an RAX gene family, an RHOXF gene family, an SATB gene family, an SEBOX gene, an SHOX gene family, an SIX gene family, a TGIF gene family, a TLX gene family, a TSHZ gene family, a UNCX gene, a VAX gene family, a VENTX gene, a VSX gene family, a ZEB gene family, a ZFHX gene family, a ZHX1 gene, etc.

The members of the DLX gene family are, for example, a DLX1 gene, a DLX2 gene, a DLX3 gene, a DLX4 gene, a DLX5 gene, and a DLX6 gene. The members of the IRX gene family are, for example, an IRX3 gene, an IRX4 gene, an IRX5 gene, and an IRX6 gene. The members of the MEIS gene family are, for example, an MEIS1 gene, an MEIS2 gene, and an MEIS3 gene. The members of the PBX gene family are, for example, a PBX1 gene, a PBX2 gene, a PBX3 gene, and a PBX4 gene. The members of the PKNOX gene family are, for example, a PKNOX1 gene and a PKNOX2 gene. The members of the ALX gene family are, for example, an ALX1 (CART1) gene, an ALX3 gene, and an ALX4 gene. The members of the BARHL gene family are, for example, a BARHL1 gene and a BARHL2 gene. The members of the BARX gene family are, for example, a BARX1 gene and a BARX2 gene. The members of the CDX gene family are, for example, a CDX1 gene, a CDX2 gene, and a CDX4 gene. The members of the CUTL gene family are, for example, a CUTL1 gene and a CUTL2 gene. The members of the DBX gene family are, for example, a DBX1 gene and a DBX2 gene. The members of the DUX gene family are, for example, a DUX1 gene, a DUX2 gene, a DUX3 gene, a DUX4 gene, a DUX5 gene, and a DUXA gene. The members of the EMX gene family are, for example, an EMX1 gene and an EMX2 gene. The members of the EN gene family are, for example, an EN1 gene and an EN2 gene. The members of the EVX gene family are, for example, an EVX1 gene and an EVX2 gene. The members of the GBX gene family are, for example, a GBX1 gene and a GBX2 gene. The members of the GSC gene family are, for example, a GSC gene and a GSC2 gene. The members of the GSX gene family are, for example, a GSX1 gene and a GSX2 gene. The members of the HMX gene family are, for example, an HMX1 gene, an HMX2 gene, and an HMX3 gene. The members of the HNF gene family are, for example, an HNF1A gene and an HNF1B gene. The members of the ISL gene family are, for example, an ISL1 gene and an ISL2 gene. The members of the LASS gene family are, for example, an LASS2 gene, an LASS3 gene, an LASS4 gene, an LASS5 gene, and an LASS6 gene. The members of the LBX gene family are, for example, an LBX1 gene and an LBX2 gene. The members of the LHX gene family are, for example, an LHX1 gene, an LHX2 gene, an LHX3 gene, an LHX4 gene, an LHX5 gene, an LHX6 gene, an LHX8 gene, and an LHX9 gene. The members of the LMX gene family are, for example, an LMX1A gene and an LMX1B gene. The members of the MEOX gene family are, for example, an MEOX1 gene and an MEOX2 gene. The members of the MSX gene family are, for example, an MSX1 gene and an MSX2 gene. The members of the NKX gene family are, for example, an NKX2-1 gene, an NKX2-2 gene, an NKX2-4 gene, an NKX2-5 gene, an NKX2-8 gene, an NKX3-1 gene, an NKX3-2 gene, an NKX6-1 gene, an NKX6-2 gene, and an NKX6-3 gene. The members of the ONECUT gene family are, for example, an ONECUT1 gene, an ONECUT2 gene, and an ONECUT3 gene. The members of the OTX gene family are, for example, an OTX1 gene and an OTX2 gene. The members of the PHOX gene family are, for example, a PHOX2A gene and a PHOX2B gene. The members of the PITX gene family are, for example, a PITX1 gene, a PITX2 gene, and a PITX3 gene. The members of the PRRX gene family are, for example, a PRRX1 gene and a PRRX2 gene. The members of the RAX gene family are, for example, an RAX gene and an RAX2 gene. The members of the RHOXF gene family are, for example, an RHOXF1 gene and an RHOXF2 gene. The members of the SATB gene family are, for example, an SATB1 gene and an SATB2 gene. The members of the SHOX gene family are, for example, an SHOX gene and an SHOX2 gene. The members of the SIX gene family are, for example, an SIX1 gene, an SIX2 gene, an SIX3 gene, an SIX4 gene, an SIX5 gene, and an SIX6 gene. The members of the TGIF gene family are, for example, a TGIF1 gene, a TGIF2 gene, a TGIF2LX gene, and a TGIF2LY gene. The members of the TLX gene family are, for example, a TLX1 gene, a TLX2 gene, and a TLX3 gene. The members of the TSHZ gene family are, for example, a TSHZ1 gene, a TSHZ2 gene, and a TSHZ3 gene. The members of the VAX gene family are, for example, a VAX1 gene and a VAX2 gene. The members of the VSX gene family are, for example, a VSX1 gene and a VSX2 gene. The members of the ZEB gene family are, for example, a ZEB1 gene and a ZEB2 gene. The members of the ZFHX gene family are, for example, a ZFHX2 gene, a ZFHX3 gene, and a ZFHX4 gene.

The members of the Hox gene family code for transcription factors and play a role in animal development etc. to determine differentiation specificity along the antero-posterior axis of the body. In vertebrates, the Hox gene family is composed of a Hoxa gene family, a Hoxb gene family, a Hoxc gene family, and a Hoxd gene family.

The members of the Hoxa gene family selected in step (A) include a Hoxa1 gene, a Hoxa2 gene, a Hoxa3 gene, a Hoxa4 gene, a Hoxa5 gene, a Hoxa6 gene, a Hoxa7 gene, a Hoxa9 gene, a Hoxa10 gene, a Hoxa11 gene, and a Hoxa13 gene. Among them, preferred are a Hoxa1 gene, a Hoxa2 gene, a Hoxa3 gene, a Hoxa4 gene, a Hoxa6 gene, a Hoxa7 gene, a Hoxa9 gene, a Hoxa10 gene, a Hoxa11 gene, and a Hoxa13 gene. The members of the Hoxb gene family selected in step (A) include a Hoxb1 gene, a Hoxb2 gene, a Hoxb3 gene, a Hoxb4 gene, a Hoxb5 gene, a Hoxb6 gene, a Hoxb7 gene, a Hoxb8 gene, a Hoxb9 gene, and a Hoxb13 gene. Among them, preferred are a Hoxb3 gene, a Hoxb4 gene, a Hoxb5 gene, a Hoxb6 gene, a Hoxb7 gene, a Hoxb8 gene, a Hoxb9 gene, and a Hoxb13 gene. The members of the Hoxc gene family selected in step (A) include a Hoxc4 gene, a Hoxc5 gene, a Hoxc6 gene, a Hoxc8 gene, a Hoxc9 gene, a Hoxc10 gene, a Hoxc11 gene, a Hoxc12 gene, and a Hoxc13 gene. Among them, preferred are a Hoxc4 gene, a Hoxc5 gene, a Hoxc6 gene, a Hoxc8 gene, a Hoxc10 gene, a Hoxc11 gene, a Hoxc12 gene, and a Hoxc13 gene. The members of the Hoxd gene family selected in step (A) include a Hoxd1 gene, a Hoxd3 gene, a Hoxd4 gene, a Hoxd8 gene, a Hoxd9 gene, a Hoxd10 gene, a Hoxd11 gene, a Hoxd12 gene, and a Hoxd13 gene. Among them, preferred are a Hoxd4 gene, a Hoxd8 gene, a Hoxd9 gene, a Hoxd10 gene, a Hoxd11 gene, a Hoxd12 gene, and a Hoxd13 gene.

The members of the Pax gene family play a central role in tissue and organ development during the embryonic stage of an animal, etc. and have a DNA-binding region called a paired domain in common.

The members of the Pax gene family selected in step (A) include a Pax1 gene, a Pax2 gene, a Pax3 gene, a Pax4 gene, a Pax5 gene, a Pax6 gene, a Pax7 gene, a Pax8 gene, and a Pax9 gene. Among them, preferred is a Pax6 gene.

In the case of using the cultured body surface tissue specimen, the gene selected in step (A) of the present invention is preferably a gene characterized by, for example, no change in the gene expression pattern before and after the culture. Examples of the gene characterized by no change in the gene expression pattern before and after the culture include a Hoxa2 gene, a Hoxa3 gene, a Hoxa5 gene, a Hoxa6 gene, a Hoxa7 gene, a Hoxa9 gene, a Hoxb3 gene, a Hoxb4 gene, a Hoxb5 gene, a Hoxb6 gene, a Hoxb7 gene, a Hoxb9 gene, a Hoxc4 gene, a Hoxc5 gene, a Hoxc6 gene, a Hoxc8 gene, a Hoxc10 gene, a Hoxc11 gene, a Hoxc12 gene, a Hoxd4 gene, a Hoxd9 gene, and a PAX6 gene. In the case where the cultured body surface tissue specimen is used, for example, it is preferable to preferentially use these genes for anatomical site determination.

Regarding the at least one gene selected in step (A) of the present invention, the upper limit of the number of genes selected is not particularly specified and is, for example, 15, 14, 13, 12, 11, 10, 9 or 8.

In step (A) of the present invention, a step of selecting genes for the combination of genes used for anatomical site determination may comprise, for example, preferentially selecting a gene whose expression level greatly differs between a high-expressing anatomical site and a low-expressing anatomical site. The selection of a gene whose expression level greatly differs between anatomical sites provides several advantageous effects. For example, the results can be easily analyzed, the positive or negative expression can be readily determined; the precision of site identification is increased; and a wide variety of body surface tissue specimens can be readily distinguished. The gene whose expression level greatly differs between anatomical sites may be, for example, a gene meeting the following requirements. In the case where the expression level of the gene in the highest-expressing site is assumed as 1.0 and other sites are divided into high-expressing sites and low-expressing sites with reference to the relative expression level threshold set at 0.1, the difference in relative expression level between a site with the lowest expression among the high-expressing sites and a site with the highest expression among the low-expressing sites is 5.0 or more, 4.0 or more, 3.0 or more, 2.0 or more, 1.0 or more, 0.08 or more, 0.06 or more, 0.04 or more, 0.02 or more, or the like. More specifically, the gene whose expression level greatly differs between anatomical sites may be, for example, a gene meeting the following requirements. In the case where the expression level of the gene in the highest-expressing site is assumed as 1.0 and other sites are divided into high-expressing sites and low-expressing sites with reference to the relative expression level threshold set at 0.1, the difference in relative expression level between a site with the lowest expression among the high-expressing sites and a site with the highest expression among the low-expressing sites is 0.08 or more. Examples of such a gene include Hoxa1, Hoxa3, Hoxa6, Hoxa13, Hoxb6, Hoxb7, Hoxb8, Hoxb9, Hoxb13, Hoxc6, Hoxc8, Hoxc10, Hoxc11, Hoxc12, Hoxd4, Hoxd9, Hoxd10 and Hoxd13 genes.

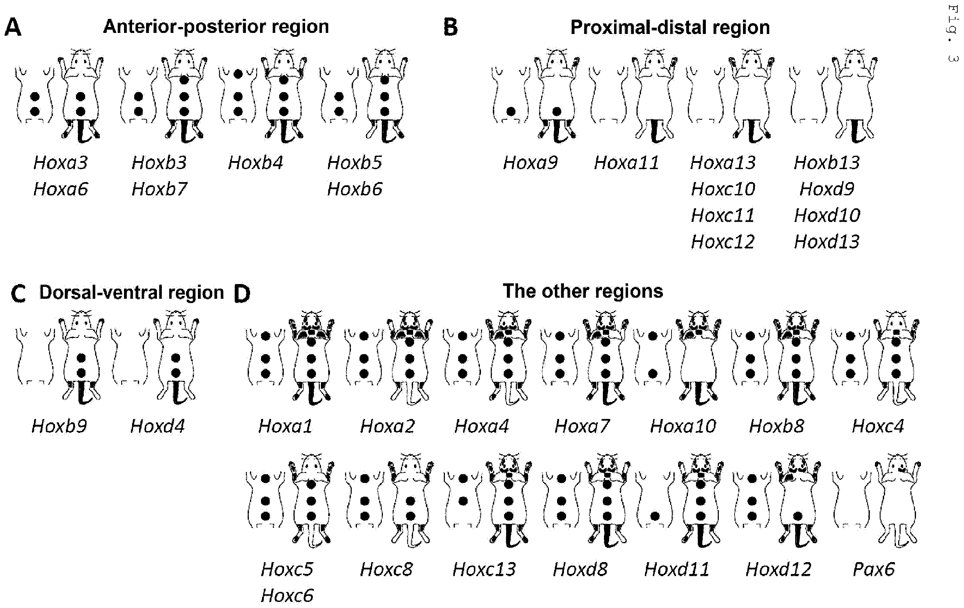

In step (A) of the present invention, a step of selecting genes for the combination of genes used for anatomical site determination may also comprise, for example, preferentially selecting a gene having a characteristic expression pattern. The gene having a characteristic expression pattern may be, for example, a gene which can be used as an index to distinguish between different anatomical sites such as between anterior and posterior regions, between proximal and distal regions, or between dorsal and ventral regions. The selection of a gene having a characteristic expression pattern provides advantageous effects, for example, the anatomical site can be roughly determined. Examples of the gene which can be used as an index to distinguish between anterior and posterior regions include the genes shown in Fig. 3A attached hereto. Examples of the gene which can be used as an index to distinguish between proximal and distal regions include the genes shown in Fig. 3B attached hereto. Examples of the gene which can be used as an index to distinguish between dorsal and ventral regions include the genes shown in Fig. 3C attached hereto.

In step (A) of the present invention, a step of selecting genes for the combination of genes used for anatomical site determination may also comprise, for example, selecting at least two kinds of genes including, for example, a gene judged to be positively expressed in an anatomical site and a gene judged to be negatively expressed therein. A combined use of genes selected in such a manner allows precise determination of anatomical sites. More specifically, for example, a combined use of the Pax6 gene and another gene negatively expressed in the cornea allows determination on whether a body surface tissue specimen has the same gene expression profile as that of the cornea.

In step (B), the expression level of the at least one gene selected in step (A) is measured in the body surface tissue specimen.

In the present invention, the method for measuring the gene expression level is not particularly limited, and a known method can be used. For example, various amplification techniques using a primer set, such as PCR, TaqMan assay, LAMP, SMAP and ICAN, can preferably be used. In particular, techniques allowing direct amplification of messenger RNA (mRNA), such as reverse transcription PCR and TaqMan assay, can preferably be used.

In the present invention, the primer set is, for example, a set of primers designed for the amplification of a member of the homeobox gene family. For example, the primer set can be designed based on the nucleotide sequence information of the selected gene obtained from known databases (e.g., GenBank etc.) . Specific examples include the primer sets shown in Table 1.

In the present invention, the order in which the expression levels of the genes are measured is not particularly limited. For example, the expression levels of the genes in the combination of genes selected in step (A) may be collectively measured, and may also be measured according to the judgement order of the genes used for stepwise anatomical site determination. The term "judgement order" used here has the same meaning as the "judgement order" used in the description for step (C) below.

In step (C), the anatomical site of origin of the body surface tissue specimen is determined based on the expression level or a combination of the expression levels measured in step (B) .

In step (C) of the present invention, the anatomical site of origin of the body surface tissue specimen can be determined, for example, by collectively using all the judgement results of the combination of the selected genes. Alternatively, the anatomical site of origin of the body surface tissue specimen can be determined in a stepwise manner according to the judgement order of the genes from the first judgement.

The judgement order of the genes in the present invention means, for example, the order in which the genes are used to sort out an anatomical site(s). In the present invention, the judgement order of the genes is represented by, for example, the first judgement, the second judgement, the third judgement, the fourth judgement, the fifth judgement, etc. In the present invention, the sorting-out of anatomical sites starts from the first judgement. The number of judgements in the judgement order can be adjusted as appropriate for the number of anatomical sites to be distinguished.

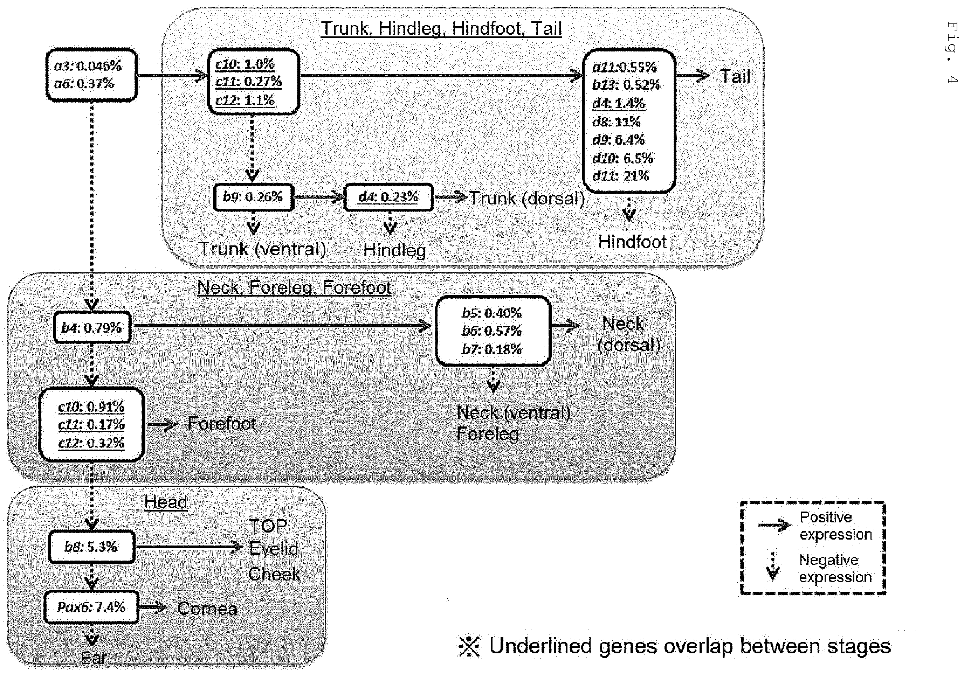

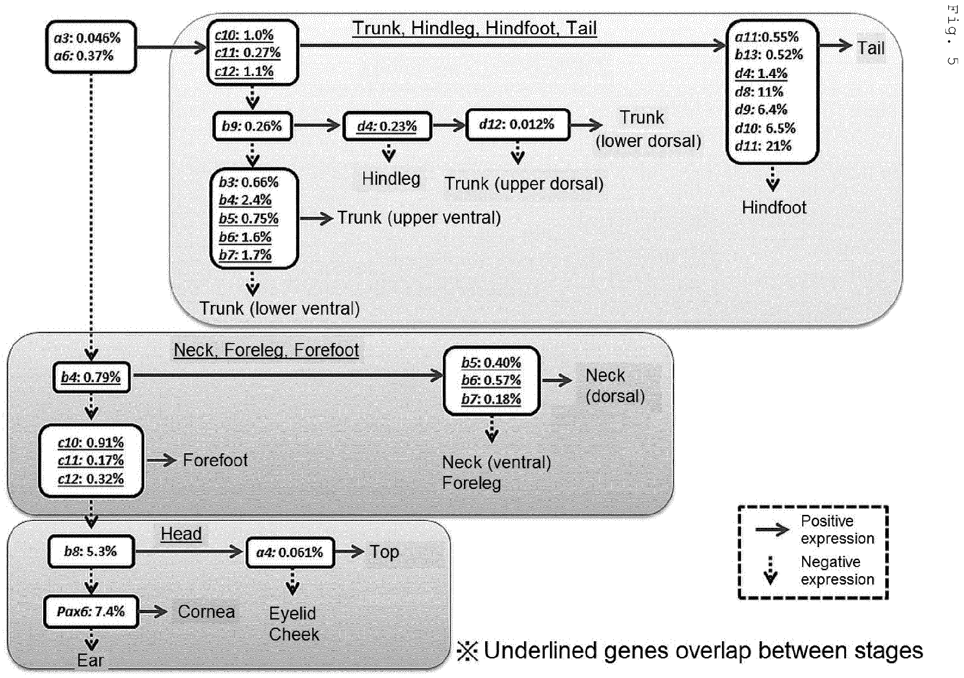

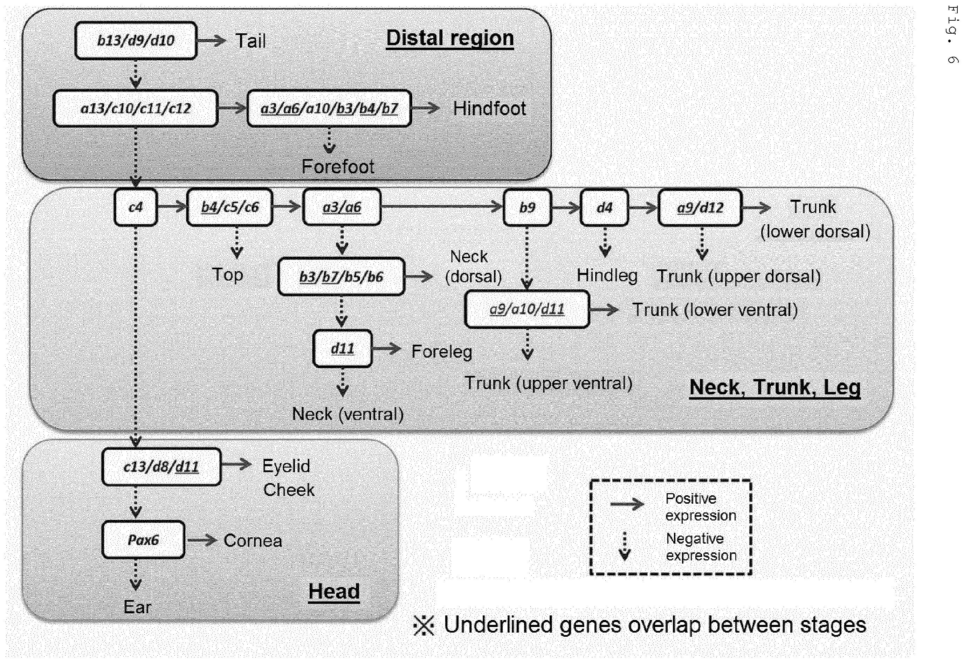

The first judgement is the first stage of anatomical site determination performed on a body surface tissue specimen. More specifically, for example, in the first judgement in the flowchart of Fig. 4 or 5 attached hereto, Hoxa3 or Hoxb6 expression is judged as positive or negative with reference to the respective reference values, and the candidate(s) is/are sorted out from all the indicated anatomical sites. In the flowchart of Fig. 6 attached hereto, Hoxb13, Hoxd9 or Hoxd10 expression is judged as positive or negative based on the analysis results of the respective expression levels, and the candidate(s) is/are sorted out from all the indicated anatomical sites.

The second judgement is the second stage of anatomical site determination following the first judgement. In the second judgement, from the anatomical sites determined to be positive for gene expression in the first judgment, positively expressing anatomical sites or negatively expressing anatomical sites are sorted out. Also, from the anatomical sites determined to be negative for gene expression in the first judgment, positively expressing anatomical sites or negatively expressing anatomical sites are sorted out. More specifically, for example, in the second judgement in the flowchart of Fig. 4 or 5 attached hereto, Hoxc10, Hoxc11 or Hoxc12 expression is judged as positive or negative with reference to the respective reference values, and the candidate(s) is/are sorted out from trunk (dorsal and ventral), hindleg, hindfoot and tail, which are the anatomical sites determined to be positive for gene expression in the first judgment. Also, Hoxb4 expression is judged as positive or negative with reference to the reference value, and the candidate(s) is/are sorted out from cornea, eyelid, ear, cheek, top, neck (dorsal and ventral), foreleg and forefoot, which are the anatomical sites determined to be negative for gene expression in the first judgment. In the flowchart of Fig. 6 attached hereto, Hoxa13, Hoxc10, Hoxc11 or Hoxc12 expression is judged as positive or negative based on the analysis results of the respective expression levels, and the candidate(s) is/are sorted out from the anatomical sites other than tail, which is the anatomical site determined to be positive for gene expression in the first judgment, that is, the anatomical sites determined to be negative for gene expression.

In the third or further judgement, as with the second judgment, for example, gene expression is further judged as positive or negative with reference to the reference value, and the candidate(s) is/are sorted out from the positively expressing anatomical sites or negatively expressing anatomical sites determined in the previous judgment.

In the present invention, the expression level of each gene may be expressed, for example, as an absolute value, a relative value or the like.

In step (C), the positive or negative expression of the selected gene can be determined based on the expression level of the gene measured in step (B). The expression level of the gene used as a reference may be, for example, an expression level preliminarily measured in a body surface tissue sample excised from an identified anatomical site of a living body etc. The expression level of the gene used as a reference may also be an expression level from the accumulated data of previous measurements in a body surface tissue sample excised from an identified anatomical site of a living body etc. The expression level of the gene used as a reference may also be an expression level publicly available from known databases etc. containing information on the expression level of a body surface tissue sample excised from an identified anatomical site of a living body etc.

In the present invention, the method for determining the positive or negative expression of the gene is not particularly limited. For example, the positive or negative expression of the gene can be determined by setting a reference value and making a comparison with the reference value. More specifically, for example, the reference value is set as a relative value to the expression level of a gene commonly expressed at similar levels in many tissues and cells (housekeeping gene) , and in the case where the expression level of the selected gene is equal to or above the reference value, the expression of the gene is judged as positive, and in the case where the expression level of the selected gene is below the reference value, the expression of the gene is judged as negative. In another example, the preliminarily measured expression level of the selected gene in a certain anatomical site is used as the reference value, and in the case where the expression level of the selected gene is equal to or above the reference value, the expression of the gene is judged as positive, and in the case where the expression level of the selected gene is below the reference value, the expression of the gene is judged as negative.

In the present invention, the housekeeping gene may be, for example, a GAPDH gene, a β-actin gene or the like.

In the present invention, the reference value is not limited as long as it can be used as a threshold for determining the positive or negative gene expression. For example, the reference value of each gene may be set as a relative expression level on the assumption that the expression level of a housekeeping gene (a GAPDH gene etc.) is 100%, and the reference value may be set at 50%, 40%, 30%, 20%, 10%, 5%, 3%, 1%, 0.8%, 0.6%, 0.4%, 0.3%, 0.2%, 0.1%, 0.05%, 0.01% or the like. Alternatively, the reference value of each gene may be set as a relative expression level on the assumption that the expression level of each gene in the highest-expressing anatomical site is 1.0, and the reference value may be set at 0.5, 0.4, 0.3, 0.2, 0.1, 0.05, 0.01 or the like. Alternatively, the reference value of each gene may be, for example, a median of the expression levels in two selected sites of the body surface tissue. In an example where the reference value of each gene is set at 0.1 on the assumption that the expression level of each gene in the highest-expressing anatomical site is 1.0, the positive or negative expression of each gene can be determined as shown in the results in Tables 2 to 5.

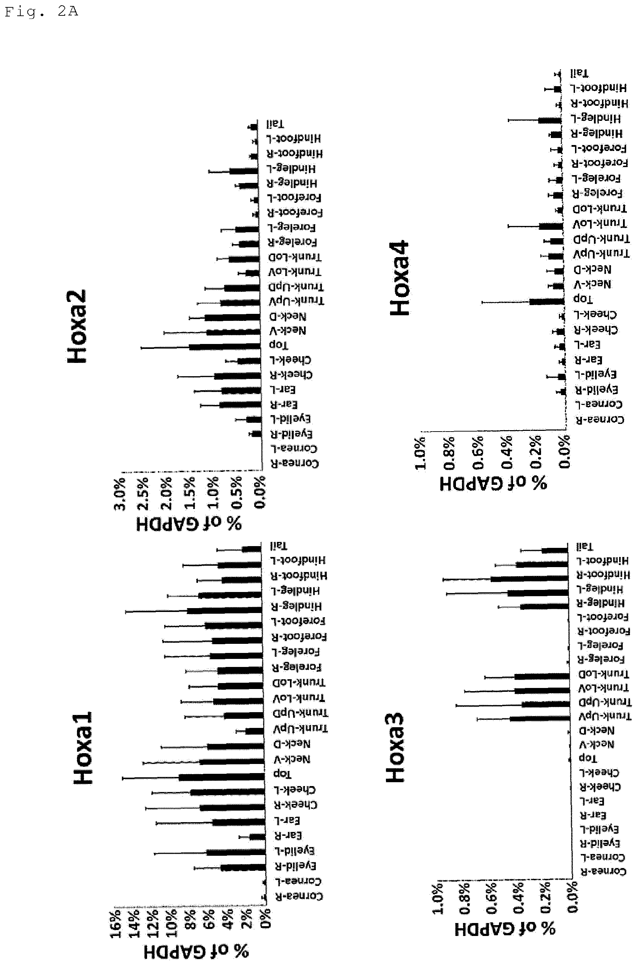

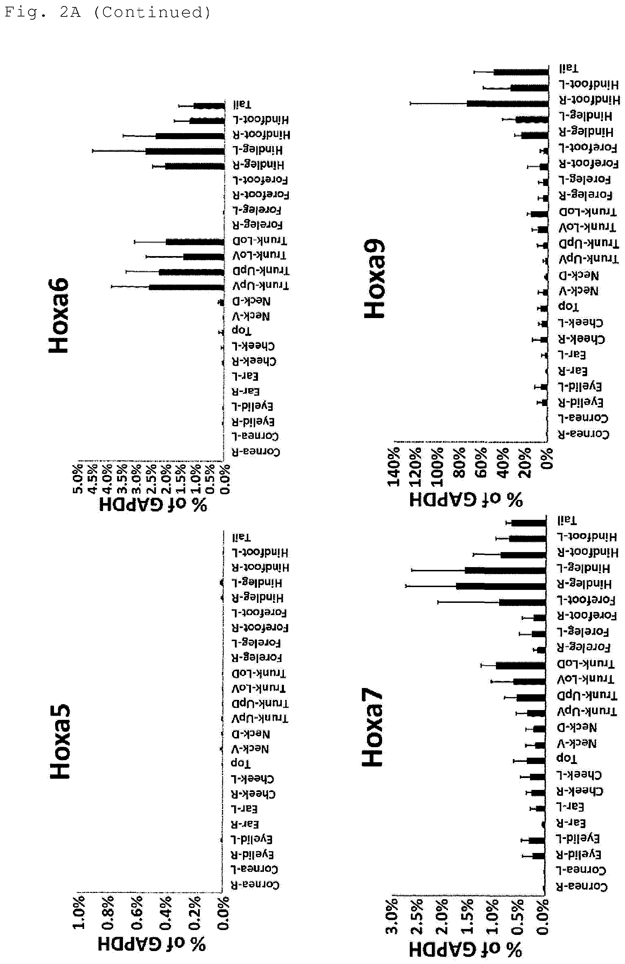

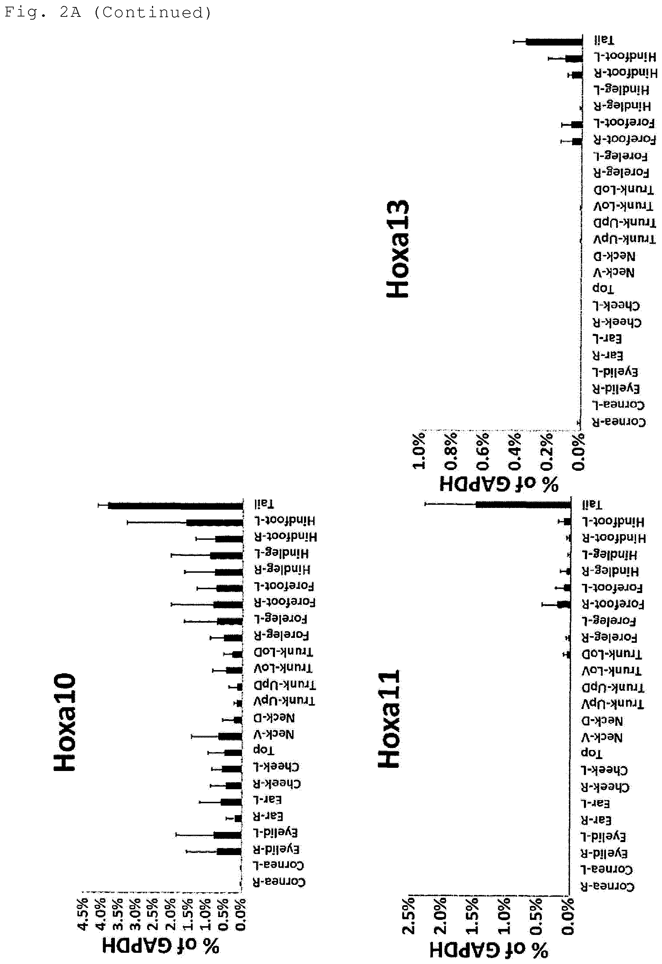

In the case where the reference value for determining the positive or negative gene expression in the present invention is a median of the expression levels in two selected sites of the body surface tissue, the two anatomical sites can be selected, for example, as follows. Based on the preliminarily measured expression levels of each gene in the plurality of anatomical sites as shown in Figs. 2A to 2D, an anatomical site with the lowest expression level among the anatomical sites to be judged as having high expression is selected as one site, and an anatomical site with the highest expression level among the anatomical sites to be judged as having low expression is selected as another site. More specifically, two anatomical sites of the body surface tissue can be selected as shown in Tables 7, 8, 10 and 11.

The median used as the reference value for determining the positive or negative gene expression can be calculated by a known method. For example, a measured gene expression level in an anatomical site with the lowest expression level among a combination of anatomical sites to be judged as having high expression is selected as one value, a measured gene expression level in an anatomical site with the highest expression level among a combination of anatomical sites to be judged as having low expression is selected as another value, and these values are used to calculate a median.

In the present invention, the median used as the reference value for determining the positive or negative gene expression can be calculated, for example, as follows. In the first judgement, based on the results of determination of the positive or negative expression in all the indicated anatomical sites shown in Tables 2 to 5, an anatomical site with the lowest expression level among the anatomical sites to be judged as having high expression is selected as one site, and an anatomical site with the highest expression level among the anatomical sites to be judged as having low expression is selected as another site. Then, the analysis results of the gene expression levels in the two sites are used to calculate a median for the first judgement.

In the second or further judgement, from the anatomical sites determined to be positive for gene expression in the first judgement, an anatomical site with the lowest expression level among the anatomical sites to be judged as having high expression is selected as one site, and an anatomical site with the highest expression level among the anatomical sites to be judged as having low expression is selected as another site. Then, the analysis results of the gene expression levels in the two sites are used to calculate a median for the second or further judgement. The thus-calculated medians can be used as the reference values to determine the positive or negative expression in individual anatomical sites. The median can be calculated by the method described in paragraph [0040].

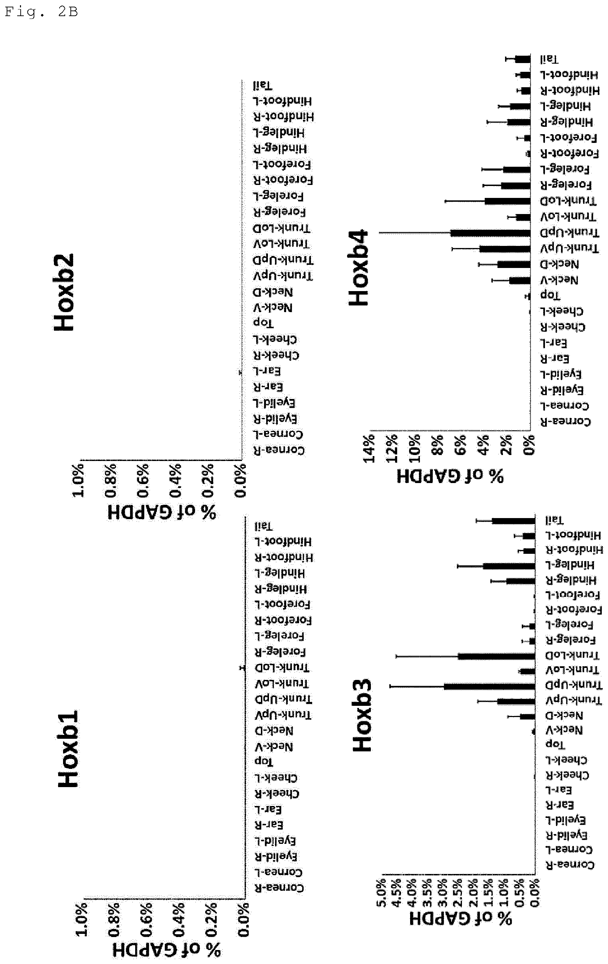

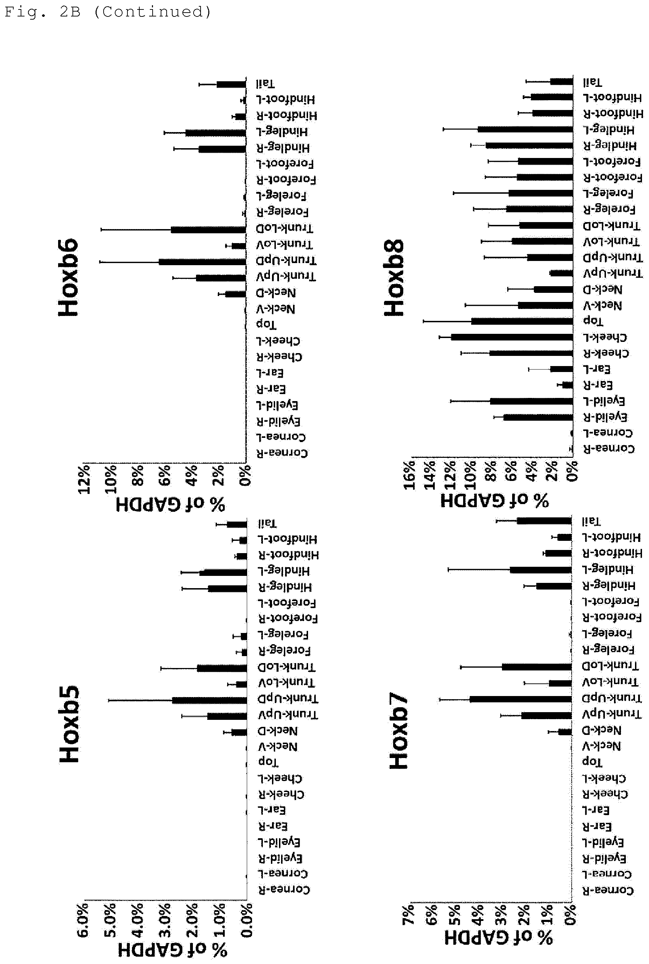

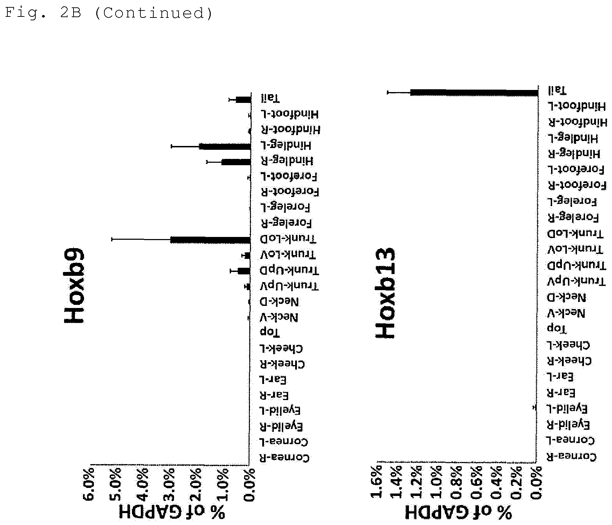

The method for calculating the median for the second or further judgement is more specifically described, for example, focusing on the Hoxb5 gene used in the third judgement in the flowchart of Fig. 4. From the anatomical sites determined to be positive for Hoxb4 gene expression with reference to the reference value in the second judgement (neck (dorsal), neck (ventral) and foreleg), the neck (dorsal) is selected as an anatomical site to be judged as having a high expression of the Hoxb5 gene, and the neck (ventral) and the foreleg are selected as anatomical sites to be judged as having a low expression of the Hoxb5 gene. This selection is based on the results of determination of the positive or negative expression shown in Table 3 and the analysis results of the gene expression level of the Hoxb5 gene shown in Fig. 2B. Further, among the neck (upper ventral and lower ventral) and the foreleg (right and left), which are anatomical sites to be judged as having a low expression of the Hoxb5 gene, the foreleg (left) is selected as the highest-expressing site. Next, a median is calculated from 3 values of the gene expression level in the neck (dorsal) (0.58%, 0.26%, 0.85% of GAPDH; n = 3) and 3 values (0.54%, 0.058%, 0.022% of GAPDH; n = 3) of the gene expression level in the foreleg (left), that is, 6 values in total, to give a value of 0.40% as a percentage relative to GAPDH expression.

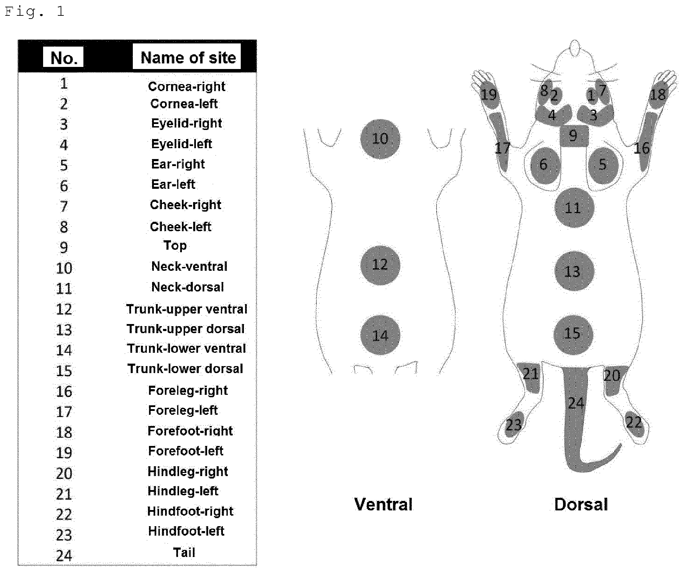

The anatomical site to be judged as having high expression is, for example, an anatomical site determined to be positive for the expression of a gene of interest based on the reference value used for the creation of Tables 2 to 5. For example, in the case where the candidate anatomical sites have been narrowed down to several sites in the determination process, the anatomical site to be judged as having high expression is, among the several candidates, an anatomical site determined to be positive for the expression in the results of determination of the positive or negative expression shown in Tables 2 to 5. For example, in the case where the candidate anatomical sites have been narrowed down to 2 sites in the determination process, the anatomical site to be judged as having high expression may be an anatomical site with the higher expression level in the analysis results of the preliminarily measured gene expression levels shown in Figs. 2A to 2D. The anatomical site to be judged as having high expression can be selected from, for example, the 24 anatomical sites shown in Fig. 1, several candidates selected from the 24 anatomical sites, and two candidates selected from the 24 anatomical sites, based on the positive or negative gene expression shown in Tables 2 to 5.

The anatomical site to be judged as having low expression is, for example, an anatomical site determined to be negative for the expression of a gene of interest based on the reference value used for the creation of Tables 2 to 5. For example, in the case where the candidate anatomical sites have been narrowed down to several sites in the determination process, the anatomical site to be judged as having low expression is, among the several candidates, an anatomical site determined to be negative for the expression in the results of determination of the positive or negative expression shown in Tables 2 to 5. For example, in the case where the candidate anatomical sites have been narrowed down to 2 sites in the determination process, the anatomical site to be judged as having low expression may be an anatomical site with the lower expression level in the analysis results of the preliminarily measured gene expression levels shown in Figs. 2A to 2D. The anatomical site to be judged as having low expression can be selected from, for example, the 24 anatomical sites shown in Fig. 1, several candidates selected from the 24 anatomical sites, and two candidates selected from the 24 anatomical sites, based on the positive or negative gene expression shown in Tables 2 to 5.

More specifically, for example in Fig. 4, in the case where the reference value of the Hoxa3 gene or the Hoxa6 gene is used, the anatomical sites to be judged as having high expression are trunk (ventral and dorsal), hindleg, hindfoot and tail as seen from the positive or negative gene expression shown in Table 2.

The combination of anatomical sites used to calculate a median as the reference value for determining the positive or negative gene expression in the present invention may vary as appropriate for the stage of the anatomical site determination process. For example, the 24 anatomical sites shown in Fig. 1 may represent one set. In the case where the candidate anatomical sites have been narrowed down to two or several sites in the final or middle stage of the anatomical site determination process, the two sites or the several sites may represent one set used to calculate a median for the next stage. More specifically, for example, in the first stage of the anatomical site determination process, in the case where a body surface tissue specimen whose gene expression profile is unknown has been judged as positive for Hoxa3 gene expression with reference to the reference value, the body surface tissue specimen is determined to be trunk (ventral and dorsal) , hindleg, hindfoot or tail in the flowchart of Fig. 4. In the second stage of the anatomical site determination process following the first stage, the trunk (ventral and dorsal), the hindleg, the hindfoot and the tail are used as a combination of anatomical sites for reference value calculation, and the these gene expression analysis results are used to calculate a median.

The median used for anatomical site determination in the present invention may be, for example, a value calculated or set based on two anatomical sites selected from the combination of anatomical sites which varies with the stage of the anatomical site determination process.

The data on gene expression levels used in calculating a median in the present invention may be, for example, the analysis results of the preliminarily measured gene expression level of each gene or the analysis results of the gene expression level of each gene measured in every experiment.

In the present invention, the method for anatomical site determination may be based on the positive or negative expression of one kind of selected gene or based on the expression pattern of two or more kinds of selected genes. For example, based on the expression pattern of the genes etc., the anatomical site of origin of a body surface tissue specimen may be judged, determined or identified, and which anatomical site has a gene expression pattern identical with or similar to that of a fabricated body surface tissue specimen may be judged, determined or identified.

In the present invention, the combination of expression levels may be, for example, any combination of positive and negative expression levels of at least two or more kinds of genes selected from the homeobox gene family.

For example, when the genes selected from the homeobox gene family are the Hoxa1 gene and the Hoxa2 gene and a combination of their expression levels are used, the anatomical site of origin of the body surface tissue specimen can be determined as follows: in the case where the expression of both the Hoxa1 and Hoxa2 genes is positive, the anatomical site of origin of the body surface tissue specimen is determined to be eyelid, ear, cheek, top, neck, trunk, foreleg or hindleg; in the case where Hoxa1 gene expression is positive and Hoxa2 gene expression is negative, the anatomical site of origin of the body surface tissue specimen is determined to be forefoot, hindfoot or tail; and in the case where the expression of both the Hoxa1 and Hoxa2 genes is negative, the anatomical site of origin of the body surface tissue specimen is determined to be cornea. Similarly, when a combination of the expression levels of any two or more selected genes is used, the anatomical site of origin of the body surface tissue specimen can be determined with reference to the results shown in Tables 2 to 5.

Preferable combinations of the expression levels used in the present invention are, for example, a combination of the expression levels of the 8 kinds of genes shown in Table 6, a combination of the expression levels of the 10 kinds of genes shown in Table 9; and a combination of the expression levels of the 11 kinds of genes shown in Table 12.

In the present invention, in the case where a flowchart or the like for anatomical site determination is created, a preferable flow is, for example, as follows. Among a combination of the selected genes, a gene that has a great difference in the expression level among anatomical sites and shows a characteristic expression pattern is preferentially subjected to expression level measurement, the expression of the gene is determined to be positive or negative, and the anatomical site is roughly determined. At the end of the flowchart, for example, a gene positively expressed in a specific anatomical site and a gene negatively expressed therein are used for anatomical site determination.

In the present invention, after the anatomical site of origin of the body surface tissue specimen is determined in step (C), the procedure from step (A) or step (B) may be repeated to determine the anatomical site of origin of the body surface tissue specimen.

### EXAMPLES

Hereinafter, the present invention will be described in detail by examples, but the present invention is not limited thereto.

### Example 1

Site-specific expression analysis of Hox gene family and Pax6 gene in mouse body surface tissue

### Excision of body surface tissue

Five-week old male C57BL/6J mice were purchased from Charles River. The mice were euthanized with carbon dioxide gas, and specimens were excised from 24 different sites of the body surface tissue. The anatomical sites of the 24 excised specimens of the body surface tissue are shown in Fig. 1. Each excised specimen of the body surface tissue was treated with 2.4 U/mL dispase (Dispase, manufactured by Thermo Fisher Scientific) in Dulbecco's Modified Eagle Medium (DMEM, manufactured by Thermo Fisher Scientific) containing penicillin and streptomycin at 4°C overnight. The epidermal tissue was separated from each treated specimen and cut into pieces in QIAzol Solution (manufactured by Qiagen) with scissors.

### qRT-PCR

Total RNA was extracted from the separated epidermal tissue with QIAzol solution according to the attached protocol. From the extracted total RNA, cDNA was synthesized with SuperScript III First-Strand Synthesis SuperMix (manufactured by Thermo Fisher Scientific) according to the attached protocol.

The cDNA was subjected to qRT-PCR to examine the expression levels of Hoxa genes (Hoxa1 to Hoxa4, Hoxa6 and Hoxa7, Hoxa9 to Hoxa11, and Hoxa13), Hoxb genes (Hoxb3 to Hoxb9 and Hoxb13), Hoxc genes (Hoxc4 to Hoxc6, Hoxc8, and Hoxc10 to Hoxc13), Hoxd genes (Hoxd4 and Hoxd8 to Hoxd13), Pax6 and K14. More specifically, qRT-PCR was performed with QuantStudio 12K Flex (manufactured by Thermo Fisher Scientific) using SYBR Premix DimerEraser (Perfect Real Time) (manufactured by TAKARA). The PCR conditions were as follows: 95°C for 10 seconds, and 45 cycles of 95°C for 5 seconds, 55°C for 30 seconds, and 72°C for 34 seconds. After PCR, melting curve analysis was performed. The primer sequences of the Hox genes used for qRT-PCR are shown in Table 1. qRT-PCR for the GAPDH gene (Mm99999915_gl), the Pax6 gene (Mm00443081_mL) and the K14 gene (Mm00516876_mL) was performed with 7500 Fast real-time PCR system (manufactured by Thermo Fisher Scientific) using TaqMan Gene Expression Assays according to the attached protocol.

**[Table 1]**

| Gene name | Primer sequence 5'→3' | | Gene name | Primer sequence 5'→3' | |
|---|---|---|---|---|---|
| Hoxa1 | GGCTGGACTACAGTGGTTGC | SEQ ID NO: 1 | Hoxc4 | AGCAACCCATAGTCTACCCTTG | SEQ ID NO: 43 |
| | TGCAAGCTTCATGACAGAGGA | SEQ ID NO: 2 | | CTTGGGCTCCCCTCCGTTAT | SEQ ID NO: 44 |
| Hoxa2 | CAGACCATTCCCAGCCTGAA | SEQ ID NO: 3 | Hoxc5 | TACCCGTGGATGACCAAACTG | SEQ ID NO: 45 |
| | CCGCTGCCATCAGCTATTTC | SEQ ID NO: 4 | | GTCTGGTAGCGCGTGTAACT | SEQ ID NO: 46 |
| Hoxa3 | TCTGAAGGCTACGTGTGCTG | SEQ ID NO: 5 | Hoxc6 | TTAGTTCTGAGCAGGGCAGG | SEQ ID NO: 47 |
| | ACCCGTAAGGGAGCACATTG | SEQ ID NO: 6 | | ACCGAGAGTAGATCTGGCGG | SEQ ID NO: 48 |
| Hoxa4 | TTGAGTCAGCCAGACAGCAC | SEQ ID NO: 7 | Hoxc8 | CCTCCGCCAACACTAACAGT | SEQ ID NO: 49 |
| | TGCATTTCCCTCTCCCCAAC | SEQ ID NO: 8 | | GTAAGTTTGTCGACCGCTGC | SEQ ID NO: 50 |
| Hoxa5 | ATGCGCAAGCTGCACATTAG | SEQ ID NO: 9 | Hoxc9 | GGCTCCAAAGTCACTGCAAAT | SEQ ID NO: 51 |
| | CAGTACTTTGGCCGCTCAGA | SEQ ID NO: 10 | | CGTAATCTGTCTCTGTCGGC | SEQ ID NO: 52 |
| Hoxa6 | CACCGACCGGAAGTACACAA | SEQ ID NO: 11 | Hoxc10 | CCAGACACCTCGGATAACGAA | SEQ ID NO: 53 |
| | CCTGCGTGGAGTTGATGAGT | SEQ ID NO: 12 | | TCCAGCGTCTGGTGTTTAGT | SEQ ID NO: 54 |
| Hoxa7 | GAACAGGGGGAATCCACACA | SEQ ID NO: 13 | Hoxc11 | AACACGAATCCCAGCTCGTC | SEQ ID NO: 55 |
| | CCAATCTCAGCAGCCCTCAC | SEQ ID NO: 14 | | AAAACTCTCGCTCCAGTTCCC | SEQ ID NO: 56 |
| Hoxa9 | GCGATGCCCCTACACAAAAC | SEQ ID NO: 15 | Hoxc12 | GACCCTGGCTCTCTGGTTTC | SEQ ID NO: 57 |
| | CTGCCTTTCGGTGAGGTTGA | SEQ ID NO: 16 | | GCTGCAACTTCGAATACGGC | SEQ ID NO: 58 |
| Hoxa10 | GGAGTGCTGGGCTGTGTTTA | SEQ ID NO: 17 | Hoxc13 | CTGGGCTCTTTCCAATGGCT | SEQ ID NO: 59 |
| | AGGCAAGCAAGACCTTAGGC | SEQ ID NO: 18 | | CTTCGGGCTGTAGAGGAACC | SEQ ID NO: 60 |
| Hoxa11 | GAAAACCTCGCTTCCTCCGA | SEQ ID NO: 19 | Hoxd1 | CCACAGCACTTTCGAGTGGA | SEQ ID NO: 61 |
| | ATAAGGGCAGCGCTTTTTGC | SEQ ID NO: 20 | | TGGTGCTGAAATTTGTGCGG | SEQ ID NO: 62 |
| Hoxa13 | CCACCTCTGGAAGTCCACTC | SEQ ID NO: 21 | Hoxd3 | CCCAAACCTGCCGAGGATG | SEQ ID NO: 63 |
| | AGCGTATTCCCGTTCGAGTT | SEQ ID NO: 22 | | TGCTGAATCTTGAGAGAGCTGG | SEQ ID NO: 64 |
| Hoxb1 | AATCGCCTTGCTCGTCAGAA | SEQ ID NO: 23 | Hoxd4 | GAAGGTGCACGTGAATTCGG | SEQ ID NO: 65 |
| | CGGACACCTTCGCTGTCTTA | SEQ ID NO: 24 | | ACTTGCTGTCTGGTGTAGGC | SEQ ID NO: 66 |
| Hoxb2 | CCCTCTGATTCTCCCTCCTGAC | SEQ ID NO: 25 | Hoxd8 | GCCCGCGAAGTTTTACGGAT | SEQ ID NO: 67 |
| | GGGAAGGAAGTCAGACACTCG | SEQ ID NO: 26 | | TATTGTACCAGCTCGGCCTC | SEQ ID NO: 68 |
| Hoxb3 | TTCGTCATGAACGGGACCAG | SEQ ID NO: 27 | Hoxd9 | GCAGCAGCAACTTGACCCAA | SEQ ID NO: 69 |
| | TATTCACATCGAGCCCCAGC | SEQ ID NO: 28 | | GGTGTAGGGACAGCGCTTTTT | SEQ ID NO: 70 |
| Hoxb4 | CTACCCCTGGATGCGCAAAG | SEQ ID NO: 29 | Hoxd10 | TGAGGTTTCCGTGTCCAGTC | SEQ ID NO: 71 |
| | GAGTGTAGGCGGTCCGAGA | SEQ ID NO: 30 | | AGCCAATTGCTGGTTGGAGT | SEQ ID NO: 72 |
| Hoxb5 | AGGGGCAGACTCCACAGATA | SEQ ID NO: 31 | Hoxd11 | CCCTTCCCATTTCCCCCAAA | SEQ ID NO: 73 |
| | CCAGGGTCTGGTAGCGAGTA | SEQ ID NO: 32 | | TGCCAACGCCCTAATCTGTT | SEQ ID NO: 74 |
| Hoxb6 | TGTTCGGAGAGACCGAGGAG | SEQ ID NO: 33 | Hoxd12 | GGCCCGCTCAACTTGAACAT | SEQ ID NO: 75 |
| | AGGGTCTGGTAGCGTGTGTA | SEQ ID NO: 34 | | TCTGCTGCTTTGTGTAGGGT | SEQ ID NO: 76 |
| Hoxb7 | CTTGGCGGCCGAGAGTAAC | SEQ ID NO: 35 | Hoxd13 | TACTGTGCCAAGGATCAGCC | SEQ ID NO: 77 |
| | CTGGTAGCGCGTGTAGGTCT | SEQ ID NO: 36 | | GCTGCAGTTTGGTGTAAGGC | SEQ ID NO: 78 |
| Hoxb8 | AGGACCTTTTAAAACTCGGTGC | SEQ ID NO: 37 | | | |
| | AGGCACGCTGTTAATTTCGC | SEQ ID NO: 38 | | | |
| Hoxb9 | GCTGGCTACGGGGACAATAA | SEQ ID NO: 39 | | | |
| | TAGGGACAGCGCTTTTTCCG | SEQ ID NO: 40 | | | |
| Hoxb13 | GAACCCACCAGGTCCATTCT | SEQ ID NO: 41 | | | |
| | TGCATACTCCCGCTCCAACT | SEQ ID NO: 42 | | | |

### Analysis data

Analysis data are shown as the mean ± standard deviation (N = 3) .

### Results

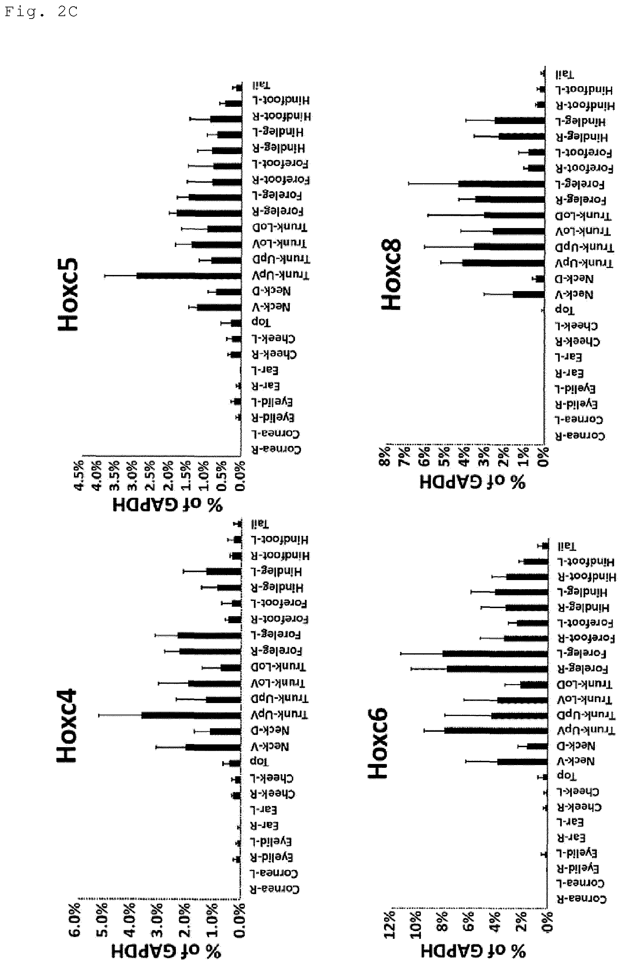

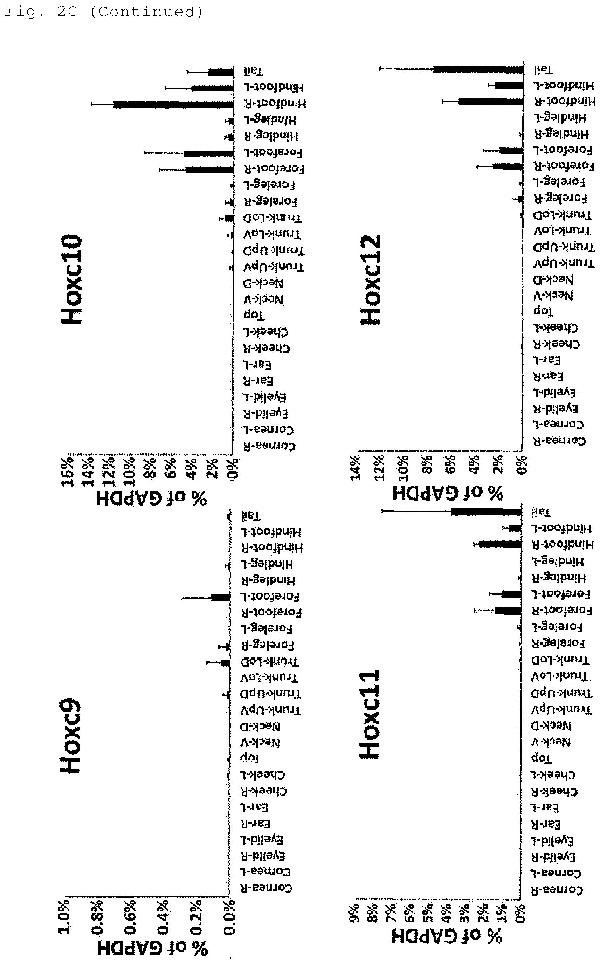

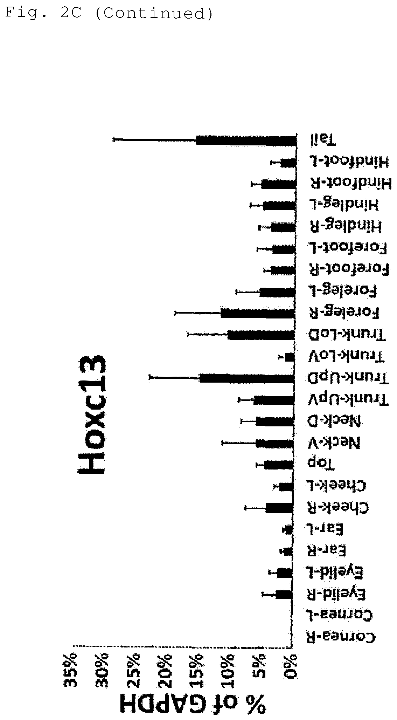

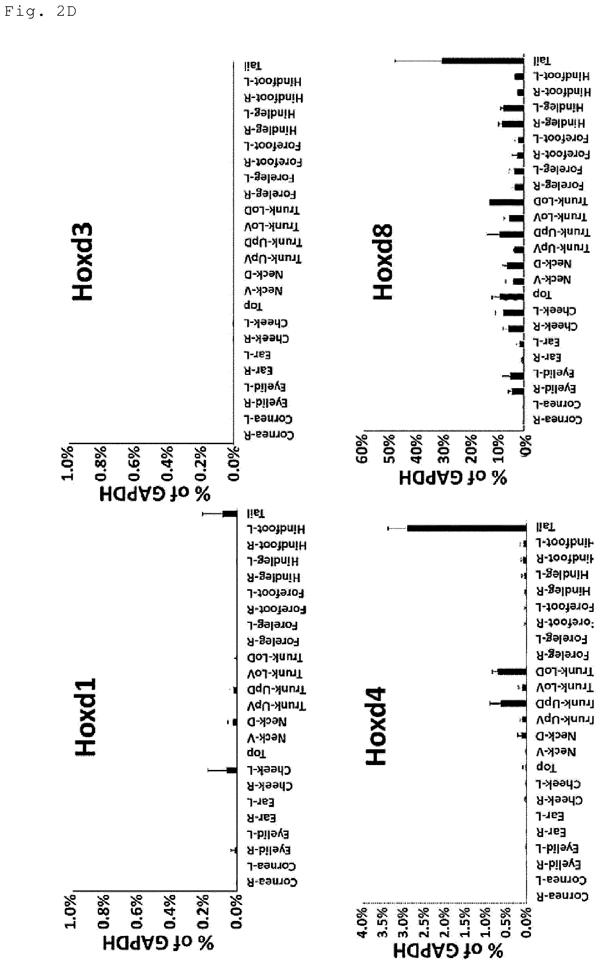

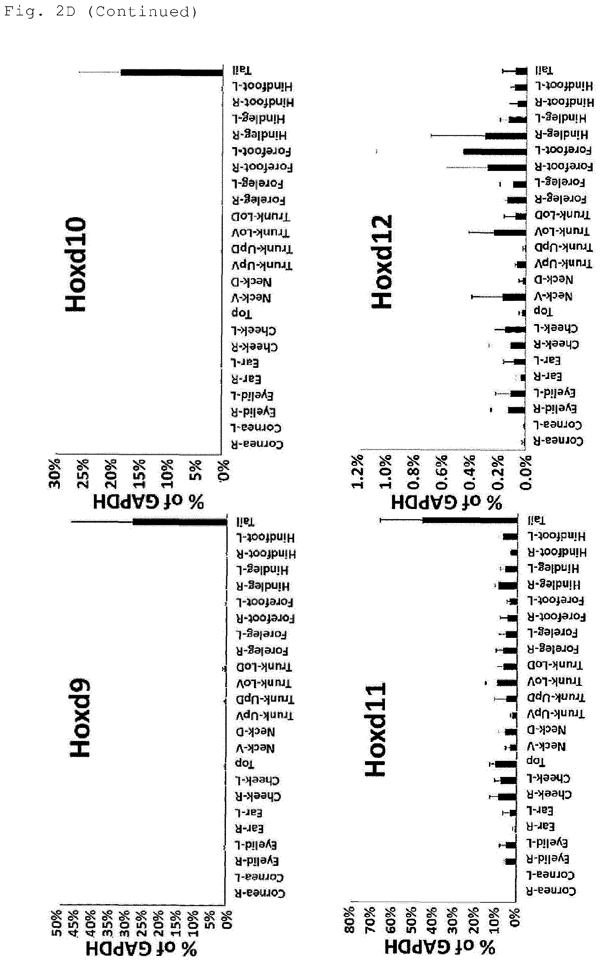

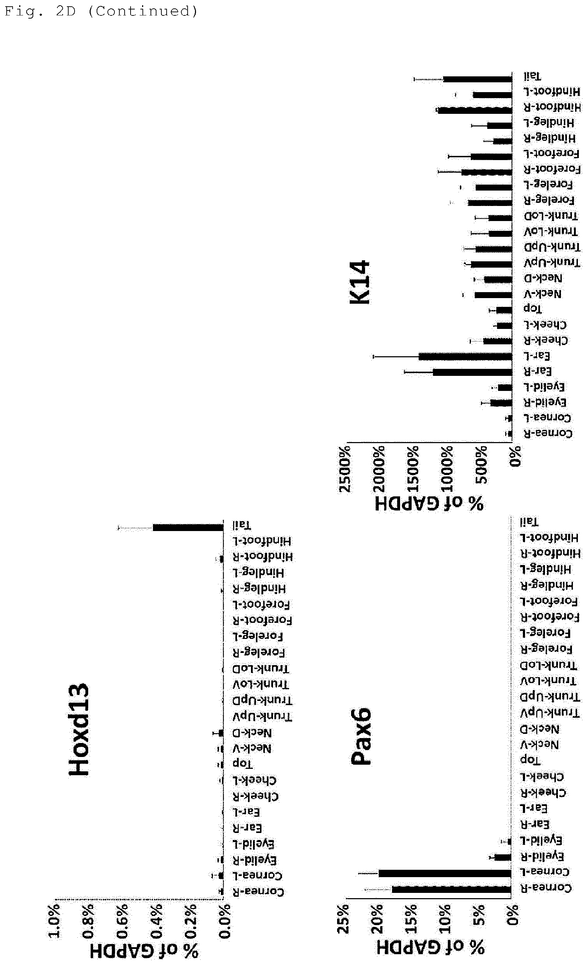

The results of the analysis of the expression levels of the Hoxa genes are shown in Fig. 2A. The results of the analysis of the expression levels of the Hoxb genes are shown in Fig. 2B. The results of the analysis of the expression levels of the Hoxc genes are shown in Fig. 2C. The results of the analysis of the expression levels of the Hoxd genes, the Pax6 gene and the K14 gene are shown in Fig. 2D.

As shown in Figs. 2A to 2D, the results for the Hoxa5 gene, the Hoxb1 gene, the Hoxb2 gene, the Hoxc9 gene, the Hoxd1 gene and the Hoxd3 gene demonstrate that, when the GAPDH gene in the mouse epidermis was assumed as 100%, the expression levels of these genes in the respective highest-expressing sites were 0.2% or less. Based on these data, the Hoxa5 gene, the Hoxb1 gene, the Hoxb2 gene, the Hoxc9 gene, the Hoxd1 gene and the Hoxd3 were judged to be negatively expressed in the mouse epidermis. These genes were not used for further analysis.

Whether the expression of each gene was positive or negative in individual sites of the mouse epidermis was determined as follows. When the expression level of a gene in the highest-expressing site was assumed as 1.0, a site with a relative expression level of 0.1 or more was judged to be positive for the expression of the gene. The judgement results of the expression of the Hoxa genes are shown in Table 2. The judgement results of the expression of the Hoxb genes are shown in Table 3. The judgement results of the expression of the Hoxc genes are shown in Table 4. The judgement results of the expression of the Hoxd genes, the Pax6 gene and the K14 gene are shown in Table 5. In Tables 2 to 5, "yes" means positive expression, and "no" means negative expression.

**[Table 2]**

| Mouse epidermis | Hoxa1 | Hoxa2 | Hoxa3 | Hoxa4 | Hoxa6 | Hoxa7 | Hoxa9 | Hoxa10 | Hoxa11 | Hoxa13 |
|---|---|---|---|---|---|---|---|---|---|---|
| Cornea-right | No | No | No | No | No | No | No | No | No | No |
| Cornea-left | No | No | No | No | No | No | No | No | No | No |
| Eyelid-right | Yes | Yes | No | Yes | No | Yes | No | Yes | No | No |
| Eyelid-left | Yes | Yes | No | Yes | No | Yes | No | Yes | No | No |
| Ear-right | Yes | Yes | No | No | No | No | No | No | No | No |
| Ear-left | Yes | Yes | No | Yes | No | Yes | No | Yes | No | No |
| Cheek-right | Yes | Yes | No | Yes | No | Yes | No | Yes | No | No |
| Cheek-left | Yes | Yes | No | No | No | Yes | No | Yes | No | No |
| Top | Yes | Yes | No | Yes | No | Yes | No | Yes | No | No |
| Neck-ventral | Yes | Yes | No | Yes | No | Yes | No | Yes | No | No |
| Neck-dorsal | Yes | Yes | No | Yes | No | Yes | No | No | No | No |
| Trunk-upper ventral | Yes | Yes | Yes | Yes | Yes | Yes | No | No | No | No |
| Trunk-upper dorsal | Yes | Yes | Yes | Yes | Yes | Yes | No | No | No | No |
| Trunk-lower ventral | Yes | Yes | Yes | Yes | Yes | Yes | Yes | Yes | No | No |
| Trunk-lower dorsal | Yes | Yes | Yes | Yes | Yes | Yes | Yes | No | No | No |
| Foreleg-right | Yes | Yes | No | Yes | No | No | No | Yes | No | No |
| Foreleg-left | Yes | Yes | No | Yes | No | Yes | No | Yes | No | No |
| Forefoot-right | Yes | No | No | Yes | No | Yes | Yes | Yes | Yes | Yes |
| Forefoot-left | Yes | No | No | Yes | No | Yes | No | Yes | No | Yes |
| Hindleg-right | Yes | Yes | Yes | Yes | Yes | Yes | Yes | Yes | No | No |
| Hindleg-left | Yes | Yes | Yes | Yes | Yes | Yes | Yes | Yes | No | No |
| Hindfoot-right | Yes | No | Yes | No | Yes | Yes | Yes | Yes | No | Yes |
| Hindfoot-left | Yes | No | Yes | Yes | Yes | Yes | Yes | Yes | No | Yes |
| Tail | Yes | No | Yes | No | Yes | Yes | Yes | Yes | Yes | Yes |

**[Table 3]**

| Mouse epidermis | Hoxb3 | Hoxb4 | Hoxb5 | Hoxb6 | Hoxb7 | Hoxb8 | Hoxb9 | Hoxb13 |
|---|---|---|---|---|---|---|---|---|
| Cornea-right | No | No | No | No | No | No | No | No |
| Cornea-left | No | No | No | No | No | No | No | No |
| Eyelid-right | No | No | No | No | No | Yes | No | No |
| Eyelid-left | No | No | No | No | No | Yes | No | No |
| Ear-right | No | No | No | No | No | No | No | No |
| Ear-left | No | No | No | No | No | Yes | No | No |
| Cheek-right | No | No | No | No | No | Yes | No | No |
| Cheek-left | No | No | No | No | No | Yes | No | No |
| Top | No | No | No | No | No | Yes | No | No |
| Neck-ventral | No | Yes | No | No | No | Yes | No | No |
| Neck-dorsal | Yes | Yes | Yes | Yes | Yes | Yes | No | No |
| Trunk-upper ventral | Yes | Yes | Yes | Yes | Yes | Yes | No | No |
| Trunk-upper dorsal | Yes | Yes | Yes | Yes | Yes | Yes | Yes | No |
| Trunk-lower ventral | Yes | Yes | Yes | Yes | Yes | Yes | No | No |
| Trunk-lower dorsal | Yes | Yes | Yes | Yes | Yes | Yes | Yes | No |
| Foreleg-right | No | Yes | No | No | No | Yes | No | No |
| Foreleg-left | No | Yes | No | No | No | Yes | No | No |
| Forefoot-right | No | No | No | No | No | Yes | No | No |
| Forefoot-left | No | No | No | No | No | Yes | No | No |
| Hindleg-right | Yes | Yes | Yes | Yes | Yes | Yes | Yes | No |
| Hindleg-left | Yes | Yes | Yes | Yes | Yes | Yes | Yes | No |
| Hindfoot-right | Yes | Yes | Yes | Yes | Yes | Yes | No | No |
| Hindfoot-left | Yes | Yes | No | No | Yes | Yes | No | No |
| Tail | Yes | Yes | Yes | Yes | Yes | Yes | Yes | Yes |

**[Table 4]**

| Mouse epidermis | Hoxc4 | Hoxc5 | Hoxc6 | Hoxc8 | Hoxc10 | Hoxc11 | Hoxc12 | Hoxc13 |
|---|---|---|---|---|---|---|---|---|
| Cornea-right | No | No | No | No | No | No | No | No |
| Cornea-left | No | No | No | No | No | No | No | No |
| Eyelid-right | No | No | No | No | No | No | No | Yes |
| Eyelid-left | No | No | No | No | No | No | No | Yes |
| Ear-right | No | No | No | No | No | No | No | No |
| Ear-left | No | No | No | No | No | No | No | No |
| Cheek-right | No | No | No | No | No | No | No | Yes |
| Cheek-left | No | No | No | No | No | No | No | Yes |
| Top | Yes | No | No | No | No | No | No | Yes |
| Neck-ventral | Yes | Yes | Yes | Yes | No | No | No | Yes |
| Neck-dorsal | Yes | Yes | Yes | No | No | No | No | Yes |
| Trunk-upper ventral | Yes | Yes | Yes | Yes | No | No | No | Yes |
| Trunk-upper dorsal | Yes | Yes | Yes | Yes | No | No | No | Yes |
| Trunk-lower ventral | Yes | Yes | Yes | Yes | No | No | No | No |
| Trunk-lower dorsal | Yes | Yes | Yes | Yes | No | No | No | Yes |
| Foreleg-right | Yes | Yes | Yes | Yes | No | No | No | Yes |
| Foreleg-left | Yes | Yes | Yes | Yes | No | No | No | Yes |
| Forefoot-right | Yes | Yes | Yes | Yes | Yes | Yes | Yes | Yes |
| Forefoot-left | No | Yes | Yes | Yes | Yes | Yes | Yes | Yes |
| Hindleg-right | Yes | Yes | Yes | Yes | No | No | No | Yes |
| Hindleg-left | Yes | Yes | Yes | Yes | No | No | No | Yes |
| Hindfoot-right | No | Yes | Yes | No | Yes | Yes | Yes | Yes |
| Hindfoot-left | No | Yes | Yes | No | Yes | Yes | Yes | Yes |
| Tail | No | No | No | No | Yes | Yes | Yes | Yes |

**[Table 5]**

| Mouse epidermis | Hoxd4 | Hoxd8 | Hoxd9 | Hoxd10 | Hoxd11 | Hoxd12 | Hoxd13 | Pax6 | K14 |
|---|---|---|---|---|---|---|---|---|---|
| Cornea-right | No | No | No | No | No | No | No | Yes | No |
| Cornea-left | No | No | No | No | No | No | No | Yes | No |
| Eyelid-right | No | Yes | No | No | Yes | Yes | No | Yes | Yes |
| Eyelid-left | No | Yes | No | No | Yes | Yes | No | No | Yes |
| Ear-right | No | No | No | No | No | No | No | No | Yes |
| Ear-left | No | No | No | No | No | Yes | No | No | Yes |
| Cheek-right | No | Yes | No | No | Yes | Yes | No | No | Yes |
| Cheek-left | No | Yes | No | No | Yes | Yes | No | No | Yes |
| Top | No | Yes | No | No | Yes | No | No | No | Yes |
| Neck-ventral | No | Yes | No | No | No | Yes | No | No | Yes |
| Neck-dorsal | No | Yes | No | No | Yes | No | No | No | Yes |
| Trunk-upper ventral | No | Yes | No | No | No | Yes | No | No | Yes |
| Trunk-upper dorsal | Yes | Yes | No | No | Yes | No | No | No | Yes |
| Trunk-lower ventral | No | Yes | No | No | Yes | Yes | No | No | Yes |
| Trunk-lower dorsal | Yes | Yes | No | No | Yes | Yes | No | No | Yes |
| Foreleg-right | No | Yes | No | No | Yes | Yes | No | No | Yes |
| Foreleg-left | No | Yes | No | No | Yes | Yes | No | No | Yes |
| Forefoot-right | No | No | No | No | Yes | Yes | No | No | Yes |
| Forefoot-left | No | No | No | No | No | Yes | No | No | Yes |
| Hindleg-right | No | Yes | No | No | Yes | Yes | No | No | Yes |
| Hindleg-left | No | Yes | No | No | Yes | Yes | No | No | Yes |
| Hindfoot-right | No | No | No | No | No | Yes | No | No | Yes |
| Hindfoot-left | No | Yes | No | No | Yes | Yes | No | No | Yes |
| Tail | Yes | Yes | Yes | Yes | Yes | Yes | Yes | No | Yes |

### Example 2

### Classification by expression pattern of Hox genes and Pax6 gene

Based on the results of the gene expression pattern in the mouse body surface tissue shown in Tables 2 to 5 of Example 1, the Hox genes and the Pax6 gene were classified into the following 4 groups:
1. genes which can determine whether the anatomical site of origin of the body surface tissue specimen is the anterior region or the posterior region;
2. genes which can determine whether the anatomical site of origin of the body surface tissue specimen is the proximal region or the distal region;
3. genes which can determine whether the anatomical site of origin of the body surface tissue specimen is the dorsal region or the ventral region; and
4. the other genes.

The results of the classification are shown in Fig. 3.

### Example 3

### Method for identifying 11 different anatomical sites of the body surface tissue using a combination of a total of 8 kinds of genes

From the Hox genes and the Pax6 gene, a total of 8 kinds of genes shown in Table 6, namely, the Hoxa3 gene or the Hoxa6 gene; the Hoxb4 gene; the Hoxb5 gene; the Hoxb8 gene; the Hoxb9 gene; the Hoxc10 gene, the Hoxc11 gene or the Hoxc12 gene; the Hoxd4 gene; and the Pax6 gene were selected.

A table regarding the selected 8 kinds of genes was created based on the gene expression analysis data shown in Figs. 2A to 2D and is shown as Table 6. Positive or negative expression of each gene in individual sites of the mouse epidermis shown in Table 6 was determined as follows. When the expression level of a gene was equal to or above the reference value, the expression of the gene was judged as positive. On the other hand, when the expression level of a gene was below the reference value, the expression of the gene was judged as negative.

In Table 6, "yes" means positive expression of the indicated gene, and "no" means negative expression of the indicated gene. The symbol "-" in Table 6 means that the expression analysis data of the indicated gene is not necessary for determining whether the anatomical site of origin of the body surface tissue specimen is the indicated site.

**[Table 6]**

| 1 | 2 | 3 | 4 | 5 | 6 | 7 | 8 | Site of body surface tissue |
|---|---|---|---|---|---|---|---|---|
| Hoxa3 or Hoxa6 | Hoxb4 | Hoxb5 | Hoxb8 | Hoxb9 | Hoxc10, Hoxc11 or Hoxc12 | Hoxd4 | Pax6 | |
| No | No | No | No | No | No | No | Yes | Cornea |
| No | No | No | Yes | No | No | No | No | Eyelid, Top, Cheek |
| No | No | No | No | No | No | No | No | Ear |
| No | Yes | No | Yes | No | No | No | No | Neck (ventral), Foreleg |
| No | Yes | Yes | Yes | No | No | No | No | Neck (dorsal) |
| Yes | Yes | Yes | Yes | No | No | No | No | Trunk (ventral) |
| Yes | Yes | Yes | Yes | Yes | No | Yes | No | Trunk (dorsal) |
| No | No | No | Yes | No | Yes | No | No | Forefoot |
| Yes | Yes | Yes | Yes | Yes | No | No | No | Hindleg |
| Yes | Yes | Yes | Yes | No | Yes | No | No | Hindfoot |
| Yes | Yes | Yes | Yes | Yes | Yes | Yes | No | Tail |
| No | - | - | - | - | - | - | - | Anterior |
| Yes | - | - | - | - | - | - | - | Posterior |
| - | - | - | - | - | No | - | - | Proximal |
| - | - | - | - | - | Yes | - | - | Distal |
| - | - | - | - | Yes | - | Yes | - | Dorsal |
| - | - | - | - | No | - | No | - | Ventral |

Based on the positive or negative expression of the 8 kinds of genes shown in Table 6, the respective reference values used for determining the anatomical site of origin of a body surface tissue specimen whose gene expression profile was unknown were calculated and are shown in Table 7. The anatomical sites (two sites) of the body surface tissue for calculating each reference value shown in Table 7 were selected from all the examined anatomical sites based on the analysis results shown in Figs. 2A to 2D and the results of determination of the positive or negative gene expression shown in Table 6.

**[Table 7]**

| Test gene | Reference value (median) (% of GAPDH) | Anatomical sites of body surface tissue (two sites) |
|---|---|---|
| Hoxa3 | 0.04 | trunk (upper dorsal) and neck (dorsal) |
| Hoxa6 | 0.37 | tail and neck (dorsal) |
| Hoxa11 | 0.13 | forefoot (right) and forefoot (left) |
| Hoxb4 | 0.79 | neck (ventral) and forefoot (left) |
| Hoxb5 | 0.35 | trunk (lower ventral) and hindfoot (left) |
| Hoxb6 | 0.47 | hindfoot (right) and hindfoot (left) |
| Hoxb7 | 0.18 | neck (dorsal) and foreleg (left) |
| Hoxb8 | 1.06 | tail and ear (right) |
| Hoxb9 | 0.22 | trunk (upper dorsal) and trunk (lower ventral) |
| Hoxb13 | 0.54 | tail and eyelid (left) |
| Hoxc10 | 0.90 | tail and trunk (lower dorsal) |
| Hoxc11 | 0.29 | forefoot (left) and foreleg (left) |
| Hoxc12 | 0.73 | forefoot (left) and foreleg (right) |
| Hoxd4 | 0.27 | trunk (upper dorsal) and neck (dorsal) |
| Hoxd8 | 3.27 | eyelid (left) and forefoot (right) |
| Hoxd9 | 6.74 | tail and trunk (lower dorsal) |
| Hoxd10 | 6.45 | tail and hindfoot (left) |
| Hoxd11 | 4.50 | eyelid (left) and ear (left) |
| Pax6 | 1.83 | eyelid (right) and eyelid (left) |

Through the analysis of the expression levels of the 8 kinds of genes using Tables 6 and 7, an anatomical site having the same characteristics as those of the body surface tissue specimen whose gene expression profile was unknown was successfully determined in an easy manner. Furthermore, in the expression analysis data of the 8 kinds of genes shown in Table 6, in particular, the expression analysis data of the single kind of gene shown in each thick frame allow easy determination on whether a body surface tissue specimen is derived from the anterior region or the posterior region; the proximal region or the distal region; or the dorsal region or the ventral region. Specifically, the expression analysis data of the Hoxa3 gene or the Hoxa6 gene allow easy determination on whether a body surface tissue specimen is derived from the anterior region or the posterior region. In addition, the expression analysis data of the Hoxc10 gene, the Hoxc11 gene or the Hoxc12 gene allow easy determination on whether a body surface tissue specimen is derived from the proximal region or the distal region. Moreover, the expression analysis data of the Hoxb9 gene or the Hoxd4 gene allow easy determination on whether a body surface tissue specimen is derived from the dorsal region or the ventral region.

With the use of the combination of the selected 8 kinds of genes, a flowchart was created (Fig. 4). The gene used at the start of the flowchart for anatomical site determination is Hoxa3 or Hoxa6, which is usable for roughly determining the anatomical site. The reference value for determining positive or negative expression of each of the selected 8 kinds of genes was calculated and is shown in Table 8. Each reference value was a median of the expression levels in the two anatomical sites (n = 3 each) of the body surface tissue shown in Table 8, that is, a median of 6 values in total. The median was calculated by a known method. The order in which the reference values shown in Table 8 are used for anatomical site determination in the flowchart is represented by 1st to 5th. The reference values for the genes assigned to "1st" were used first.

The reference value of Hoxa3 or Hoxa6 at the start of the flowchart was calculated as follows. Based on the results of gene expression analysis in all the examined anatomical sites, trunk, hindleg, hindfoot and tail were selected as anatomical sites positive for Hoxa3 or Hoxa6 expression, the other sites were selected as anatomical sites negative for Hoxa3 or Hoxa6 expression, and two anatomical sites of the body surface tissue were selected for calculation. Next, in the case where the expression of the Hoxa3 gene or the Hoxa6 gene had been judged as positive, the trunk, the hindleg, the hindfoot and the tail were selected as anatomical sites for reference value calculation. Among these sites, the hindfoot and the tail were selected as anatomical sites positive for gene expression, the trunk (ventral and dorsal) and the hindleg were selected as anatomical sites negative for gene expression, and the reference value of the Hoxc10 gene, the Hoxc11 gene or the Hoxc12 gene was calculated. In the case where the expression of the Hoxa3 gene or the Hoxa6 gene had been judged to be negative, neck (dorsal and ventral) and foreleg were selected as anatomical sites positive for Hoxb4 gene expression, the others were selected as anatomical sites negative for Hoxb4 gene expression, and the reference value was calculated. Subsequent determination was similarly performed.

**[Table 8]**

| Test gene | Reference value (median) (% of GAPDH) | Anatomical sites of body surface tissue (two sites) | Judgement order |
|---|---|---|---|
| Hoxa3 | 0.046 | top and tail | 1st |
| Hoxa6 | 0.37 | neck (dorsal) and tail | |
| Hoxa11 | 0.55 | hindfoot (left) and tail | 3rd |
| Hoxb4 | 0.79 | forefoot (left) and neck (ventral) | 2nd |
| Hoxb5 | 0.40 | foreleg (left) and neck (dorsal) | 3rd |
| Hoxb6 | 0.57 | foreleg (right) and neck (dorsal) | |
| Hoxb7 | 0.18 | foreleg (left) and neck (dorsal) | |
| Hoxb8 | 5.3 | ear (left) and eyelid (right) | 4th |
| Hoxb9 | 0.26 | trunk (lower ventral) and trunk (upper dorsal) | 3rd |
| Hoxb13 | 0.52 | hindfoot (right) and tail | 3rd |
| Hoxd8 | 11 | hindfoot (left) and tail | |
| Hoxd9 | 6.4 | hindfoot (right) and tail | |
| Hoxd10 | 6.5 | hindfoot (left) and tail | |
| Hoxd11 | 21 | hindfoot (left) and tail | |
| Pax6 | 7.4 | ear (right) and cornea (right) | 5th |
| Hoxc10 | 1.0 | trunk (lower dorsal) and tail | 2nd |
| (the case of determination for trunk, hindleg, hindfoot and tail) | | | |
| Hoxc11 | 0.27 | hindleg (right) and hindfoot (left) | |
| (the case of determination for trunk, hindleg, hindfoot and tail) | | | |
| Hoxc12 | 1.1 | hindleg (right) and hindfoot (left) | |
| (the case of determination for trunk, hindleg, hindfoot and tail) | | | |
| Hoxc10 | 0.91 | eyelid (right) and forefoot (right) | 3rd |
| (the case of determination for cornea, eyelid, ear, cheek, top and forefoot) | | | |
| Hoxc11 | 0.17 | eyelid (right) and forefoot (left) | |
| (the case of determination for cornea, eyelid, ear, cheek, top and forefoot) | | | |
| Hoxc12 | 0.32 | top and forefoot (left) | |
| (the case of determination for cornea, eyelid, ear, cheek, top and forefoot) | | | |
| Hoxd4 | 1.4 | hindfoot (right) and tail | 3rd |
| (the case of determination for hindfoot and tail) | | | |
| Hoxd4 | 0.23 | hindleg (left) and trunk (upper dorsal) | 4th |
| (the case of determination for trunk (dorsal) and hindleg) | | | |

With the use of Table 8 and Fig. 4, a maximum of 11 sites were successfully determined through the analysis of the expression levels of a minimum of 8 kinds of genes.

### Example 4

### Method for identifying 14 different anatomical sites of the body surface tissue using a combination of a total of 10 kinds of genes

From the Hox genes and the Pax6 gene, a total of 10 kinds of genes shown in Table 9, namely, the Hoxa3 gene or the Hoxa6 gene; the Hoxa4 gene; the Hoxb4 gene; the Hoxb5 gene, the Hoxb6 gene, or the Hoxb7 gene; the Hoxb8 gene; the Hoxb9 gene; the Hoxc10 gene, the Hoxc11 gene or the Hoxc12 gene; the Hoxd4 gene; the Hoxd12 gene; and the Pax6 gene were selected for combination.

A table regarding the selected 10 kinds of genes was created based on the gene expression analysis data shown in Figs. 2A to 2D and is shown as Table 9. Positive or negative expression of each gene in individual sites of the mouse epidermis shown in Table 9 was determined as follows. When the expression level of a gene was equal to or above the reference value, the expression of the gene was judged as positive. On the other hand, when the expression level of a gene was below the reference value, the expression of the gene was judged as negative.

In Table 9, "yes" means positive expression of the indicated gene, and "no" means negative expression of the indicated gene. The symbol "-" in Table 9 means that the expression analysis data of the indicated gene is not necessary for determining whether the anatomical site of origin of the body surface tissue specimen is the indicated site.

**[Table 9]**

| 1 | 2 | 3 | 4 | 5 | 6 | 7 | 8 | 9 | 10 | Site of body surface tissue |
|---|---|---|---|---|---|---|---|---|---|---|
| Hoxa3 or Hoxa6 | Hoxa4 | Hoxb4 | Hoxb5, Hoxb6 or Hoxb7 | Hoxb8 | Hoxb9 | Hoxc10, Hoxc11 or Hoxc12 | Hoxd4 | Hoxd12 | Pax6 | |
| No | No | No | No | No | No | No | No | No | Yes | Cornea |
| No | Yes | No | No | Yes | No | No | No | Yes | No | Eyelid, Cheek |
| No | Yes | No | No | No | No | No | No | Yes | No | Ear |
| No | Yes | No | No | Yes | No | No | No | No | No | Top |
| No | Yes | Yes | No | Yes | No | No | No | Yes | No | Neck (ventral), Foreleg |
| No | Yes | Yes | Yes | Yes | No | No | No | No | No | Neck (dorsal) |
| Yes | Yes | Yes | Yes | Yes | No | No | No | Yes | No | Trunk (upper ventral) |
| Yes | Yes | Yes | Yes | Yes | Yes | No | Yes | No | No | Trunk (upper dorsal) |
| Yes | Yes | Yes | Yes | Yes | No | No | No | Yes | No | Trunk (lower ventral) |
| Yes | Yes | Yes | Yes | Yes | Yes | No | Yes | Yes | No | Trunk (lower dorsal) |
| No | Yes | No | No | Yes | No | Yes | No | Yes | No | Forefoot |
| Yes | Yes | Yes | Yes | Yes | Yes | No | No | Yes | No | Hindleg |
| Yes | Yes | Yes | Yes | Yes | No | Yes | No | Yes | No | Hindfoot |
| Yes | No | Yes | Yes | Yes | Yes | Yes | Yes | Yes | No | Tail |
| No | - | No | No | - | - | - | - | - | - | Anterior |
| Yes | - | Yes | Yes | - | - | - | - | - | - | Posterior |
| - | - | - | - | - | - | No | - | - | - | Proximal |
| - | - | - | - | - | - | Yes | - | - | - | Distal |
| - | - | - | - | - | Yes | - | Yes | - | - | Dorsal |
| - | - | - | - | - | No | - | No | - | - | Ventral |

Based on the positive or negative expression of the 10 kinds of genes shown in Table 9, the respective reference values used for determining the anatomical site of origin of a body surface tissue specimen whose gene expression profile was unknown were calculated and are shown in Table 10. The anatomical sites (two sites) of the body surface tissue for calculating each reference value shown in Table 10 were selected from all the examined anatomical sites based on the analysis results shown in Figs. 2A to 2D and the results of determination of the positive or negative gene expression shown in Table 9.

**[Table 10]**

| Test gene | Reference value (median) (% of GAPDH) | Anatomical sites of body surface tissue (two sites) |
|---|---|---|
| Hoxa3 | 0.04 | trunk (upper dorsal) and neck (dorsal) |
| Hoxa4 | 0.021 | eyelid (left) and tail |
| Hoxa6 | 0.37 | tail and neck (dorsal) |
| Hoxa11 | 0.13 | forefoot (right) and forefoot (left) |
| Hoxb3 | 0.31 | neck (dorsal) and foreleg (right) |
| Hoxb4 | 0.79 | neck (ventral) and forefoot (left) |
| Hoxb5 | 0.35 | trunk (lower ventral) and hindfoot (left) |
| Hoxb6 | 0.47 | hindfoot (right) and hindfoot (left) |
| Hoxb7 | 0.18 | neck (dorsal) and foreleg (left) |
| Hoxb8 | 1.06 | tail and ear (right) |
| Hoxb9 | 0.22 | trunk (upper dorsal) and trunk (lower ventral) |
| Hoxb13 | 0.54 | tail and eyelid (left) |
| Hoxc10 | 0.90 | tail and trunk (lower dorsal) |
| Hoxc11 | 0.29 | forefoot (left) and foreleg (left) |
| Hoxc12 | 0.73 | forefoot (left) and foreleg (right) |
| Hoxd4 | 0.27 | trunk (upper dorsal) and neck (dorsal) |
| Hoxd8 | 3.27 | eyelid (left) and forefoot (right) |
| Hoxd9 | 6.74 | tail and trunk (lower dorsal) |
| Hoxd10 | 6.45 | tail and hindfoot (left) |
| Hoxd11 | 4.50 | eyelid (left) and ear (left) |
| Hoxd12 | 0.032 | ear (left) and ear (right) |
| Pax6 | 1.83 | eyelid (right) and eyelid (left) |

Through the analysis of the expression levels of the 10 kinds of genes using Tables 9 and 10, an anatomical site having the same characteristics as those of the body surface tissue specimen whose gene expression profile was unknown was successfully determined in an easy manner. Furthermore, in the expression analysis data of the 10 kinds of genes shown in Table 9, in particular, the expression analysis data of the single kind of gene shown in each thick frame allow easy determination on whether a body surface tissue specimen is derived from the anterior region or the posterior region; the proximal region or the distal region; or the dorsal region or the ventral region. Specifically, the expression analysis data of the Hoxa3 gene, the Hoxa6 gene, the Hoxb4 gene, the Hoxb5 gene, the Hoxb6 gene or the Hoxb7 gene allow easy determination on whether a body surface tissue specimen is derived from the anterior region or the posterior region. In addition, the expression analysis data of the Hoxc10 gene, the Hoxc11 gene or the Hoxc12 gene allow easy determination on whether a body surface tissue specimen is derived from the proximal region or the distal region. Moreover, the expression analysis data of the Hoxb9 gene or the Hoxd4 gene allow easy determination on whether a body surface tissue specimen is derived from the dorsal region or the ventral region.

With the use of the combination of the selected 10 kinds of genes, a flowchart was created (Fig. 5). The reference values of the genes described in the flowchart were calculated in the same manner as described in Example 3 and are shown in Table 11. The two anatomical sites of the body surface tissue for reference value calculation were selected in the same manner as described in Example 3 and used. The order in which the reference values shown in Table 11 are used for anatomical site determination in the flowchart is represented by 1st to 5th. The reference values for the genes assigned to "1st" were used first.

**[Table 11]**

| Test gene | Reference value (median) (% of GAPDH) | Anatomical sites of body surface tissue (two sites) | Judgement order |
|---|---|---|---|
| Hoxa3 | 0.046 | top and tail | 1st |
| Hoxa4 | 0.061 | cheek (right) and top | 5th |
| Hoxa6 | 0.37 | neck (dorsal) and tail | 1st |
| Hoxa11 | 0.55 | hindfoot (left) and tail | 3rd |
| Hoxb3 | 0.66 | trunk (lower ventral) and trunk (upper ventral) | 4th |
| Hoxb8 | 5.3 | ear (left) and eyelid (right) | 4th |
| Hoxb9 | 0.26 | trunk (lower ventral) and trunk (upper dorsal) | 3rd |
| Hoxb13 | 0.52 | hindfoot (right) and tail | 3rd |
| Hoxd8 | 11 | hindfoot (left) and tail | |
| Hoxd9 | 6.4 | hindfoot (right) and tail | |
| Hoxd10 | 6.5 | hindfoot (left) and tail | |
| Hoxd11 | 21 | hindfoot (left) and tail | |
| Hoxd12 | 0.012 | trunk (upper dorsal) and trunk (lower dorsal) | 5th |
| Pax6 | 7.4 | ear (right) and cornea (right) | 5th |
| Hoxb4 | 2.4 | trunk (lower ventral) and trunk (upper ventral) | 4th |
| (the case of determination for trunk (upper ventral) and trunk (lower ventral)) | | | |
| Hoxb5 | 0.75 | trunk (lower ventral) and trunk (upper ventral) | |
| (the case of determination for trunk (upper ventral) and trunk (lower ventral)) | | | |
| Hoxb6 | 1.6 | trunk (lower ventral) and trunk (upper ventral) | |
| (the case of determination for trunk (upper ventral) and trunk (lower ventral)) | | | |
| Hoxb7 | 1.7 | trunk (lower ventral) and trunk (upper ventral) | |
| (the case of determination for trunk (upper ventral) and trunk (lower ventral)) | | | |
| Hoxb4 | 0.79 | forefoot (left) and neck (ventral) | 2nd |
| (the case of determination for neck, foreleg and forefoot) | | | |
| Hoxb5 | 0.40 | foreleg (left) and neck (dorsal) | 3rd |
| (the case of determination for neck (dorsal), neck (ventral) and foreleg) | | | |
| Hoxb6 | 0.57 | foreleg (right) and neck (dorsal) | |
| (the case of determination for neck (dorsal), neck (ventral) and foreleg) | | | |
| Hoxb7 | 0.18 | foreleg (left) and neck (dorsal) | |
| (the case of determination for neck (dorsal), neck (ventral) and foreleg) | | | |
| Hoxc10 | 1.0 | trunk (lower dorsal) and tail | 2nd |
| (the case of determination for trunk, hindleg, hindfoot and tail) | | | |
| Hoxc11 | 0.27 | hindleg (right) and hindfoot (left) | |
| (the case of determination for trunk, hindleg, hindfoot and tail) | | | |
| Hoxc12 | 1.1 | hindleg (right) and hindfoot (left) | |
| (the case of determination for trunk, hindleg, hindfoot and tail) | | | |
| Hoxc10 | 0.91 | eyelid (right) and forefoot (right) | 3rd |
| (the case of determination for cornea, eyelid, ear, cheek, top and forefoot) | | | |
| Hoxc11 | 0.17 | eyelid (right) and forefoot (left) | |
| (the case of determination for cornea, eyelid, ear, cheek, top and forefoot) | | | |
| Hoxc12 | 0.32 | top and forefoot (left) | |
| (the case of determination for cornea, eyelid, ear, cheek, top and forefoot) | | | |
| Hoxd4 | 1.4 | hindfoot (right) and tail | 3rd |
| (the case of determination for hindfoot and tail) | | | |
| Hoxd4 | 0.23 | hindleg (left) and trunk (upper dorsal) | 4th |
| (the case of determination for trunk (dorsal) and hindleg) | | | |

With the use of Table 11 and Fig. 5, a maximum of 14 sites were successfully determined through the analysis of the expression levels of a minimum of 10 kinds of genes.

### Example 5

### Method for identifying 15 different anatomical sites of the body surface tissue using a combination of a total of 11 kinds of genes

From the Hox genes and the Pax6 gene, a total of 11 kinds of genes shown in Table 12, namely, the Hoxa3 gene or the Hoxa6 gene; the Hoxa9 gene; the Hoxa13 gene, the Hoxc10 gene, the Hoxc11 gene or the Hoxc12 gene; the Hoxb3 gene or the Hoxb7 gene; the Hoxb4 gene; the Hoxb9 gene; the Hoxb13 gene, the Hoxd9 gene or the Hoxd10 gene; the Hoxc4 gene; the Hoxd4 gene; the Hoxd11 gene; and the Pax6 gene were selected for combination. A table of the expression of each gene in individual anatomical sites of the body surface tissue was created based on the determination results shown in Tables 2 to 5 and is shown as Table 12. In Table 12, "yes" means positive expression of the indicated gene, and "no" means negative expression of the indicated gene. The symbol "-" in Table 12 means that the expression analysis data of the indicated gene is not necessary for determining whether the anatomical site of origin of the body surface tissue specimen is the indicated site.

**[Table 12]**

| 1 | 2 | 3 | 4 | 5 | 6 | 7 | 8 | 9 | 10 | 11 | Site of body surface tissue |
|---|---|---|---|---|---|---|---|---|---|---|---|
| Hoxa3 or Hoxa6 | Hoxa9 | Hoxa13, Hoxc10, Hoxc11 or Hoxc12 | Hoxb3 or Hoxb7 | Hoxb4 | Hoxb9 | Hoxb13, Hoxd9 or Hoxd10 | Hoxc4 | Hoxd4 | Hoxd11 | Pax6 | |
| No | No | No | No | No | No | No | No | No | No | Yes | Cornea |
| No | No | No | No | No | No | No | No | No | Yes | No | Eyelid, Cheek |
| No | No | No | No | No | No | No | No | No | No | No | Ear |
| No | No | No | No | No | No | No | Yes | No | Yes | No | Top |
| No | No | No | No | Yes | No | No | Yes | No | No | No | Neck (ventral) |
| No | No | No | Yes | Yes | No | No | Yes | No | Yes | No | Neck (dorsal) |
| Yes | No | No | Yes | Yes | No | No | Yes | No | No | No | Trunk |
| | | | | | | | | | | | (upper ventral) |
| Yes | No | No | Yes | Yes | Yes | No | Yes | Yes | Yes | No | Trunk |
| | | | | | | | | | | | (upper dorsal) |
| Yes | Yes | No | Yes | Yes | No | No | Yes | No | Yes | No | Trunk |
| | | | | | | | | | | | (lower ventral) |
| Yes | Yes | No | Yes | Yes | Yes | No | Yes | Yes | Yes | No | Trunk |
| | | | | | | | | | | | (lower dorsal) |
| No | No | No | No | Yes | No | No | Yes | No | Yes | No | Foreleg |
| No | Yes | Yes | No | No | No | No | Yes | No | Yes | No | Forefoot |
| Yes | Yes | Yes | Yes | Yes | Yes | No | Yes | No | Yes | No | Hindleg |
| Yes | Yes | Yes | Yes | Yes | No | No | No | No | Yes | No | Hindfoot |
| Yes | Yes | Yes | Yes | Yes | Yes | Yes | No | Yes | Yes | No | Tail |
| No | - | - | No | No | - | - | - | - | - | - | Anterior |
| Yes | - | - | Yes | Yes | - | - | - | - | - | - | Posterior |
| - | No | No | - | - | - | No | - | - | - | - | Proximal |
| - | Yes | Yes | - | - | - | Yes | - | - | - | - | Distal |
| - | - | - | - | - | Yes | - | - | Yes | - | - | Dorsal |
| - | - | - | - | - | No | - | - | No | - | - | Ventral |

With the use of Table 12, a maximum of 15 sites were successfully determined through the analysis of the expression levels of a minimum of 11 kinds of genes. Furthermore, in the expression analysis data of the 11 kinds of genes shown in Table 12, in particular, the expression analysis data of the single kind of gene shown in each thick frame allow easy determination on whether a body surface tissue specimen is derived from the anterior region or the posterior region; the proximal region or the distal region; or the dorsal region or the ventral region. Specifically, the expression analysis data of the Hoxa3 gene, the Hoxa6 gene, the Hoxb3 gene, the Hoxb4 gene or the Hoxb7 gene allow easy determination on whether a body surface tissue specimen is derived from the anterior region or the posterior region. In addition, the expression analysis data of the Hoxa9 gene, the Hoxa13 gene, the Hoxb13 gene, the Hoxc10 gene, the Hoxc11 gene, the Hoxc12 gene, the Hoxd9 gene or the Hoxd10 gene allow easy determination on whether a body surface tissue specimen is derived from the proximal region or the distal region. Moreover, the expression analysis data of the Hoxb9 gene or the Hoxd4 gene allow easy determination on whether a body surface tissue specimen is derived from the dorsal region or the ventral region.

A flowchart used for determination of 15 anatomical sites of origin of the body surface tissue specimen was created based on the expression analysis data of a minimum of 11 kinds of genes shown in Table 12 and is shown in Fig. 6. Following the flowchart of Fig. 6, whether the anatomical site was the proximal region or the distal region was first determined, and then, whether the anatomical site was neck, trunk, leg, or head was determined using the Hoxc4 gene.

## Claims

1. A method for determining an anatomical site of origin of a body surface tissue specimen, the method comprising the steps of:
(A) selecting at least one gene of a homeobox gene family;
(B) measuring the expression level of the at least one gene selected in the above (A) in the body surface tissue specimen; and
(C) determining the anatomical site of origin of the body surface tissue specimen based on the expression level or a combination of the expression levels measured in the above (B) .

2. The method according to claim 1, wherein the homeobox gene family consists of a Hox gene family and a Pax6 gene.

3. The method according to claim 2, wherein the HOX gene family consists of Hoxa1 to Hoxa4, Hoxa6, Hoxa7, Hoxa9 to Hoxa11, and Hoxa13; Hoxb3 to Hoxb9, and Hoxb13; Hoxc4 to Hoxc6, Hoxc8, and Hoxc10 to Hoxc13; and Hoxd4, and Hoxd8 to Hoxd13.

4. The method according to any one of claims 1 to 3, wherein the anatomical site of origin of the body surface tissue specimen is at least one site selected from cornea-right, cornea-left, eyelid-right, eyelid-left, ear-right, ear-left, cheek-right, cheek-left, top, neck-ventral, neck-dorsal, trunk-upper ventral, trunk-upper dorsal, trunk-lower ventral, trunk-lower dorsal, foreleg-right, foreleg-left, forefoot-right, forefoot-left, hindleg-right, hindleg-left, hindfoot-right, hindfoot-left, and tail.

5. The method according to any one of claims 1 to 4, wherein, in step (A), the genes specified in Table 6, namely, the following 8 kinds of genes:
1: Hoxa3 or Hoxa6,
2: Hoxb4,
3: Hoxb5,
4: Hoxb8,
5: Hoxb9,
6: Hoxc10, Hoxc11, or Hoxc12,
7: Hoxd4, and
8: Pax6
are selected, and
wherein, in step (C), the expression of each selected gene is determined to be positive or negative, and the anatomical site of origin of the body surface tissue specimen is determined with reference to the gene expression patterns specified in Table 6.

6. The method according to any one of claims 1 to 4, wherein, in step (A), the genes specified in Table 9, namely, the following 10 kinds of genes:
1: Hoxa3 or Hoxa6,
2: Hoxa4,
3: Hoxb4,
4: Hoxb5, Hoxb6, or Hoxb7,
5: Hoxb8,
6: Hoxb9,
7: Hoxc10, Hoxc11, or Hoxc12,
8: Hoxd4,
9: Hoxd12, and
10: Pax6
are selected, and
wherein, in step (C), the expression of each selected gene is determined to be positive or negative, and the anatomical site of origin of the body surface tissue specimen is determined with reference to the gene expression patterns specified in Table 9.

7. The method according to claim 5 or 6, wherein a first anatomical site determination comprises determining the anatomical site of origin of the body surface tissue specimen with reference to the expression pattern of at least one gene selected from the group consisting of Hoxa3 and Hoxa6.

8. The method according to any one of claims 1 to 4, wherein, in step (A), the genes specified in Table 12, namely, the following 11 kinds of genes:
1: Hoxa3 or Hoxa6,
2: Hoxa9,
3: Hoxa13, Hoxc10, Hoxc11, or Hoxc12,
4: Hoxb3 or Hoxb7,
5: Hoxb4,
6: Hoxb9,
7: Hoxb13, Hoxd9, or Hoxd10,
8: Hoxc4,
9: Hoxd4,
10: Hoxd11, and
11: Pax6
are selected, and
wherein, in step (C), the expression of each selected gene is determined to be positive or negative, and the anatomical site of origin of the body surface tissue specimen is determined with reference to the gene expression patterns specified in Table 12.

9. The method according to claim 8, wherein a first anatomical site determination comprises determining the anatomical site of origin of the body surface tissue specimen with reference to the expression pattern of at least one gene selected from the group consisting of Hoxb13, Hoxd9 and Hoxd10.

10. The method according to any one of claims 1 to 4, wherein, in step (A), one gene selected from the group consisting of the Hoxa3 gene, the Hoxa6 gene, the Hoxb3 gene, the Hoxb4 gene, the Hoxb5 gene, the Hoxb6 gene and the Hoxb7 gene is selected, and
wherein, in step (C), the expression of the selected gene is determined to be positive or negative; and in the case of positive expression of the selected gene, the anatomical site of origin of the body surface tissue specimen is determined to be a posterior region, and in the case of negative expression of the selected gene, the anatomical site of origin of the body surface tissue specimen is determined to be an anterior region.

11. The method according to any one of claims 1 to 4, wherein, in step (A), one gene selected from the group consisting of the Hoxa9 gene, the Hoxa11 gene, the Hoxa13 gene, the Hoxb13 gene, the Hoxc10 gene, the Hoxc11 gene, the Hoxc12 gene, the Hoxd9 gene, the Hoxd10 gene, and the Hoxd13 gene is selected, and
wherein, in step (C), the expression of the selected gene is determined to be positive or negative; and in the case of positive expression of the selected gene, the anatomical site of origin of the body surface tissue specimen is determined to be a distal region, and in the case of negative expression of the selected gene, the anatomical site of origin of the body surface tissue specimen is determined to be a proximal region.

12. The method according to any one of claims 1 to 4, wherein, in step (A), the Hoxb9 gene or the Hoxd4 gene is selected, and
wherein, in step (C), the expression of the selected gene is determined to be positive or negative; and in the case of positive expression of the selected gene, the anatomical site of origin of the body surface tissue specimen is determined to be a dorsal region, and in the case of negative expression of the selected gene, the anatomical site of origin of the body surface tissue specimen is determined to be a ventral region.

13. The method according to claim 4, wherein the anatomical site of origin of the body surface tissue specimen is determined in any of the following manners:
(1) in step (A), the Hoxa3 gene or the Hoxa6 gene is selected, and
in step (C), the expression of the selected gene is determined to be positive or negative; and in the case of positive expression of the selected gene, the anatomical site of origin of the body surface tissue specimen is determined to be trunk, hindleg, hindfoot or tail as set forth in claim 4, and in the case of negative expression of the selected gene, the anatomical site of origin of the body surface tissue specimen is determined to be any of the other anatomical sites as set forth in claim 4;
(2) in step (A), the Hoxb3 gene or the Hoxb7 gene is selected, and
in step (C), the expression of the selected gene is determined to be positive or negative; and in the case of positive expression of the selected gene, the anatomical site of origin of the body surface tissue specimen is determined to be neck-dorsal, trunk, hindleg, hindfoot or tail as set forth in claim 4, and in the case of negative expression of the selected gene, the anatomical site of origin of the body surface tissue specimen is determined to be any of the other anatomical sites as set forth in claim 4;
(3) in step (A), the Hoxb4 gene is selected, and
in step (C), the expression of the selected gene is determined to be positive or negative; and in the case of positive expression of the selected gene, the anatomical site of origin of the body surface tissue specimen is determined to be neck, trunk, foreleg, hindleg, hindfoot or tail as set forth in claim 4, and in the case of negative expression of the selected gene, the anatomical site of origin of the body surface tissue specimen is determined to be any of the other anatomical sites as set forth in claim 4;
(4) in step (A), the Hoxb5 gene or the Hoxb6 gene is selected, and
in step (C), the expression of the selected gene is determined to be positive or negative; and in the case of positive expression of the selected gene, the anatomical site of origin of the body surface tissue specimen is determined to be neck-dorsal, trunk, hindleg, hindfoot-right or tail as set forth in claim 4, and in the case of negative expression of the selected gene, the anatomical site of origin of the body surface tissue specimen is determined to be any of the other anatomical sites as set forth in claim 4;
(5) in step (A), the Hoxa9 gene is selected, and
in step (C), the expression of the selected gene is determined to be positive or negative; and in the case of positive expression of the selected gene, the anatomical site of origin of the body surface tissue specimen is determined to be trunk-lower, forefoot-right, hindleg, hindfoot or tail as set forth in claim 4, and in the case of negative expression of the selected gene, the anatomical site of origin of the body surface tissue specimen is determined to be any of the other anatomical sites as set forth in claim 4;
(6) in step (A), the Hoxa11 gene is selected, and
in step (C), the expression of the selected gene is determined to be positive or negative; and in the case of positive expression of the selected gene, the anatomical site of origin of the body surface tissue specimen is determined to be forefoot-right or tail as set forth in claim 4, and in the case of negative expression of the selected gene, the anatomical site of origin of the body surface tissue specimen is determined to be any of the other anatomical sites as set forth in claim 4;
(7) in step (A), the Hoxa13 gene, the Hoxc10 gene, the Hoxc11 gene, or the Hoxc12 gene is selected, and
in step (C), the expression of the selected gene is determined to be positive or negative; and in the case of positive expression of the selected gene, the anatomical site of origin of the body surface tissue specimen is determined to be forefoot, hindfoot or tail as set forth in claim 4, and in the case of negative expression of the selected gene, the anatomical site of origin of the body surface tissue specimen is determined to be any of the other anatomical sites as set forth in claim 4;
(8) in step (A), the Hoxb13 gene, the Hoxd9 gene, or the Hoxd10 gene is selected, and
in step (C), the expression of the selected gene is determined to be positive or negative; and in the case of positive expression of the selected gene, the anatomical site of origin of the body surface tissue specimen is determined to be tail as set forth in claim 4, and in the case of negative expression of the selected gene, the anatomical site of origin of the body surface tissue specimen is determined to be any of the other anatomical sites as set forth in claim 4;
(9) in step (A), the Hoxd13 gene is selected, and
in step (C), the expression of the selected gene is determined to be positive or negative; and in the case of positive expression of the selected gene, the anatomical site of origin of the body surface tissue specimen is determined to be tail as set forth in claim 4, and in the case of negative expression of the selected gene, the anatomical site of origin of the body surface tissue specimen is determined to be any of the other anatomical sites as set forth in claim 4;
(10) in step (A), the Hoxb9 gene is selected, and
in step (C), the expression of the selected gene is determined to be positive or negative; and in the case of positive expression of the selected gene, the anatomical site of origin of the body surface tissue specimen is determined to be trunk-upper dorsal, trunk-lower dorsal, hindleg or tail as set forth in claim 4, and in the case of negative expression of the selected gene, the anatomical site of origin of the body surface tissue specimen is determined to be any of the other anatomical sites as set forth in claim 4;
(11) in step (A), the Hoxd4 gene is selected, and
in step (C), the expression of the selected gene is determined to be positive or negative; and in the case of positive expression of the selected gene, the anatomical site of origin of the body surface tissue specimen is determined to be trunk-upper dorsal, trunk-lower dorsal or tail as set forth in claim 4, and in the case of negative expression of the selected gene, the anatomical site of origin of the body surface tissue specimen is determined to be any of the other anatomical sites as set forth in claim 4;
(12) in step (A), the Hoxa1 gene is selected, and
in step (C), the expression of the selected gene is determined to be positive or negative; and in the case of negative expression of the selected gene, the anatomical site of origin of the body surface tissue specimen is determined to be cornea as set forth in claim 4, and in the case of positive expression of the selected gene, the anatomical site of origin of the body surface tissue specimen is determined to be any of the other anatomical sites as set forth in claim 4;
(13) in step (A), the Hoxa2 gene is selected, and
in step (C), the expression of the selected gene is determined to be positive or negative; and in the case of negative expression of the selected gene, the anatomical site of origin of the body surface tissue specimen is determined to be cornea, forefoot, hindfoot or tail as set forth in claim 4, and in the case of positive expression of the selected gene, the anatomical site of origin of the body surface tissue specimen is determined to be any of the other anatomical sites as set forth in claim 4;
(14) in step (A), the Hoxa4 gene is selected, and
in step (C), the expression of the selected gene is determined to be positive or negative; and in the case of negative expression of the selected gene, the anatomical site of origin of the body surface tissue specimen is determined to be cornea, ear-right, cheek-left, hindfoot-right or tail as set forth in claim 4, and in the case of positive expression of the selected gene, the anatomical site of origin of the body surface tissue specimen is determined to be any of the other anatomical sites as set forth in claim 4;
(15) in step (A), the Hoxa7 gene is selected, and
in step (C), the expression of the selected gene is determined to be positive or negative; and in the case of negative expression of the selected gene, the anatomical site of origin of the body surface tissue specimen is determined to be cornea, ear-right or foreleg-right as set forth in claim 4, and in the case of positive expression of the selected gene, the anatomical site of origin of the body surface tissue specimen is determined to be any of the other anatomical sites as set forth in claim 4;
(16) in step (A), the Hoxa10 gene is selected, and
in step (C), the expression of the selected gene is determined to be positive or negative; and in the case of negative expression of the selected gene, the anatomical site of origin of the body surface tissue specimen is determined to be cornea, ear-right, neck-dorsal, trunk-upper or trunk-lower dorsal as set forth in claim 4, and in the case of positive expression of the selected gene, the anatomical site of origin of the body surface tissue specimen is determined to be any of the other anatomical sites as set forth in claim 4;
(17) in step (A), the Hoxb8 gene is selected, and
in step (C), the expression of the selected gene is determined to be positive or negative; and in the case of negative expression of the selected gene, the anatomical site of origin of the body surface tissue specimen is determined to be cornea or ear-right as set forth in claim 4, and in the case of positive expression of the selected gene, the anatomical site of origin of the body surface tissue specimen is determined to be any of the other anatomical sites as set forth in claim 4;
(18) in step (A), the Hoxc4 gene is selected, and
in step (C), the expression of the selected gene is determined to be positive or negative; and in the case of negative expression of the selected gene, the anatomical site of origin of the body surface tissue specimen is determined to be cornea, eyelid, ear, cheek, forefoot-left, hindfoot or tail as set forth in claim 4, and in the case of positive expression of the selected gene, the anatomical site of origin of the body surface tissue specimen is determined to be any of the other anatomical sites as set forth in claim 4;
(19) in step (A), the Hoxc5 gene or the Hoxc6 gene is selected, and
in step (C), the expression of the selected gene is determined to be positive or negative; and in the case of negative expression of the selected gene, the anatomical site of origin of the body surface tissue specimen is determined to be cornea, eyelid, ear, cheek, top or tail as set forth in claim 4, and in the case of positive expression of the selected gene, the anatomical site of origin of the body surface tissue specimen is determined to be any of the other anatomical sites as set forth in claim 4;
(20) in step (A), the Hoxc8 gene is selected, and
in step (C), the expression of the selected gene is determined to be positive or negative; and in the case of negative expression of the selected gene, the anatomical site of origin of the body surface tissue specimen is determined to be cornea, eyelid, ear, cheek, top, neck-dorsal, hindfoot or tail as set forth in claim 4, and in the case of positive expression of the selected gene, the anatomical site of origin of the body surface tissue specimen is determined to be any of the other anatomical sites as set forth in claim 4;
(21) in step (A), the Hoxc13 gene is selected, and
in step (C), the expression of the selected gene is determined to be positive or negative; and in the case of negative expression of the selected gene, the anatomical site of origin of the body surface tissue specimen is determined to be cornea, ear or trunk-lower ventral as set forth in claim 4, and in the case of positive expression of the selected gene, the anatomical site of origin of the body surface tissue specimen is determined to be any of the other anatomical sites as set forth in claim 4;
(22) in step (A), the Hoxd8 gene is selected, and
in step (C), the expression of the selected gene is determined to be positive or negative; and in the case of negative expression of the selected gene, the anatomical site of origin of the body surface tissue specimen is determined to be cornea, ear, forefoot or hindfoot-right as set forth in claim 4, and in the case of positive expression of the selected gene, the anatomical site of origin of the body surface tissue specimen is determined to be any of the other anatomical sites as set forth in claim 4;
(23) in step (A), the Hoxd11 gene is selected, and
in step (C), the expression of the selected gene is determined to be positive or negative; and in the case of negative expression of the selected gene, the anatomical site of origin of the body surface tissue specimen is determined to be cornea, ear, neck-ventral, trunk-upper ventral, forefoot-left or hindfoot-right as set forth in claim 4, and in the case of positive expression of the selected gene, the anatomical site of origin of the body surface tissue specimen is determined to be any of the other anatomical sites as set forth in claim 4;
(24) in step (A), the Hoxd12 gene is selected, and
in step (C), the expression of the selected gene is determined to be positive or negative; and in the case of negative expression of the selected gene, the anatomical site of origin of the body surface tissue specimen is determined to be cornea, ear-right, top, neck-dorsal or trunk-upper dorsal as set forth in claim 4, and in the case of positive expression of the selected gene, the anatomical site of origin of the body surface tissue specimen is determined to be any of the other anatomical sites as set forth in claim 4; and
(25) in step (A), the Pax6 gene is selected, and
in step (C), the expression of the selected gene is determined to be positive or negative; and in the case of positive expression of the selected gene, the anatomical site of origin of the body surface tissue specimen is determined to be cornea or eyelid-right as set forth in claim 4, and in the case of negative expression of the selected gene, the anatomical site of origin of the body surface tissue specimen is determined to be any of the other anatomical sites as set forth in claim 4.

14. The method according to any one of claims 1 to 13, wherein the body surface tissue specimen is epidermis or cornea.

15. The method according to any one of claims 1 to 14, wherein the epidermis or the cornea is prepared by excision or fabrication, or by excision or fabrication followed by culture.


SEQUENCE LISTING

<110>  Osaka University

<120>  A method for determining anatomical region of surface tissues

<130>  O11F6102

<150>  JP 2017-013414
<151>  2017-01-27

<160>  78

<170>  PatentIn version 3.1

<210>  1
<211>  20
<212>  DNA
<213>  Artificial

<220>
<223>  Primer

<400>  1
ggctggacta cagtggttgc                                                   20


<210>  2
<211>  21
<212>  DNA
<213>  Artificial

<220>
<223>  Primer

<400>  2
tgcaagcttc atgacagagg a                                                 21


<210>  3
<211>  20
<212>  DNA
<213>  Artificial

<220>
<223>  Primer

<400>  3
cagaccattc ccagcctgaa                                                   20


<210>  4
<211>  20
<212>  DNA
<213>  Artificial

<220>
<223>  Primer

<400>  4
ccgctgccat cagctatttc                                                   20

<210>  5
<211>  20
<212>  DNA
<213>  Artificial

<220>
<223>  Primer

<400>  5
tctgaaggct acgtgtgctg                                                    20


<210>  6
<211>  20
<212>  DNA
<213>  Artificial

<220>
<223>  Primer

<400>  6
acccgtaagg gagcacattg                                                    20


<210>  7
<211>  20
<212>  DNA
<213>  Artificial

<220>
<223>  Primer

<400>  7
ttgagtcagc cagacagcac                                                    20


<210>  8
<211>  20
<212>  DNA
<213>  Artificial

<220>
<223>  Primer

<400>  8
tgcatttccc tctccccaac                                                    20


<210>  9
<211>  20
<212>  DNA
<213>  Artificial

<220>
<223>  Primer

<400>  9
atgcgcaagc tgcacattag                                                    20


<210>  10
<211>  20
<212>  DNA

<213>  Artificial

<220>
<223>  Primer

<400>  10
cagtactttg gccgctcaga                                                    20


<210>  11
<211>  20
<212>  DNA
<213>  Artificial

<220>
<223>  Primer

<400>  11
caccgaccgg aagtacacaa                                                    20


<210>  12
<211>  20
<212>  DNA
<213>  Artificial

<220>
<223>  Primer

<400>  12
cctgcgtgga gttgatgagt                                                    20


<210>  13
<211>  20
<212>  DNA
<213>  Artificial

<220>
<223>  Primer

<400>  13
gaacaggggg aatccacaca                                                    20


<210>  14
<211>  20
<212>  DNA
<213>  Artificial

<220>
<223>  Primer

<400>  14
ccaatctcag cagccctcac                                                    20


<210>  15
<211>  20
<212>  DNA
<213>  Artificial

<220>

<223>  Primer

<400>  15
gcgatgcccc tacacaaaac                                                    20


<210>  16
<211>  20
<212>  DNA
<213>  Artificial

<220>
<223>  Primer

<400>  16
ctgcctttcg gtgaggttga                                                    20


<210>  17
<211>  20
<212>  DNA
<213>  Artificial

<220>
<223>  Primer

<400>  17
ggagtgctgg gctgtgttta                                                    20


<210>  18
<211>  20
<212>  DNA
<213>  Artificial

<220>
<223>  Primer

<400>  18
aggcaagcaa gaccttaggc                                                    20


<210>  19
<211>  20
<212>  DNA
<213>  Artificial

<220>
<223>  Primer

<400>  19
gaaaacctcg cttcctccga                                                    20


<210>  20
<211>  20
<212>  DNA
<213>  Artificial

<220>
<223>  Primer

<400>  20

ataaggcag cgctttttgc                                                    20


<210>  21
<211>  20
<212>  DNA
<213>  Artificial

<220>
<223>  Primer

<400>  21
ccacctctgg aagtccactc                                                    20


<210>  22
<211>  20
<212>  DNA
<213>  Artificial

<220>
<223>  Primer

<400>  22
agcgtattcc cgttcgagtt                                                    20


<210>  23
<211>  20
<212>  DNA
<213>  Artificial

<220>
<223>  Primer

<400>  23
aatcgccttg ctcgtcagaa                                                    20


<210>  24
<211>  20
<212>  DNA
<213>  Artificial

<220>
<223>  Primer

<400>  24
cggacacctt cgctgtctta                                                    20


<210>  25
<211>  22
<212>  DNA
<213>  Artificial

<220>
<223>  Primer

<400>  25
ccctctgatt ctccctcctg ac                                                 22

<210>  26
<211>  21
<212>  DNA
<213>  Artificial

<220>
<223>  Primer

<400>  26
gggaaggaag tcagacactc g                                                21


<210>  27
<211>  20
<212>  DNA
<213>  Artificial

<220>
<223>  Primer

<400>  27
ttcgtcatga acgggaccag                                                  20


<210>  28
<211>  20
<212>  DNA
<213>  Artificial

<220>
<223>  Primer

<400>  28
tattcacatc gagccccagc                                                  20


<210>  29
<211>  20
<212>  DNA
<213>  Artificial

<220>
<223>  Primer

<400>  29
ctacccctgg atgcgcaaag                                                  20


<210>  30
<211>  19
<212>  DNA
<213>  Artificial

<220>
<223>  Primer

<400>  30
gagtgtaggc ggtccgaga                                                   19


<210>  31
<211>  20
<212>  DNA

<213>  Artificial

<220>
<223>  Primer

<400>  31
aggggcagac tccacagata                                                    20


<210>  32
<211>  20
<212>  DNA
<213>  Artificial

<220>
<223>  Primer

<400>  32
ccagggtctg gtagcgagta                                                    20


<210>  33
<211>  20
<212>  DNA
<213>  Artificial

<220>
<223>  Primer

<400>  33
tgttcggaga gaccgaggag                                                    20


<210>  34
<211>  20
<212>  DNA
<213>  Artificial

<220>
<223>  Primer

<400>  34
agggtctggt agcgtgtgta                                                    20


<210>  35
<211>  19
<212>  DNA
<213>  Artificial

<220>
<223>  Primer

<400>  35
cttggcggcc gagagtaac                                                     19


<210>  36
<211>  20
<212>  DNA
<213>  Artificial

<220>

<223>  Primer

<400>  36
ctggtagcgc gtgtaggtct                                                    20


<210>  37
<211>  22
<212>  DNA
<213>  Artificial

<220>
<223>  Primer

<400>  37
aggacctttt aaaactcggt gc                                                 22


<210>  38
<211>  20
<212>  DNA
<213>  Artificial

<220>
<223>  Primer

<400>  38
aggcacgctg ttaatttcgc                                                    20


<210>  39
<211>  20
<212>  DNA
<213>  Artificial

<220>
<223>  Primer

<400>  39
gctggctacg gggacaataa                                                    20


<210>  40
<211>  20
<212>  DNA
<213>  Artificial

<220>
<223>  Primer

<400>  40
tagggacagc gctttttccg                                                    20


<210>  41
<211>  20
<212>  DNA
<213>  Artificial

<220>
<223>  Primer

<400>  41

gaacccacca ggtccattct                                                    20

<210>  42
<211>  20
<212>  DNA
<213>  Artificial

<220>
<223>  Primer

<400>  42
tgcatactcc cgctccaact                                                    20

<210>  43
<211>  22
<212>  DNA
<213>  Artificial

<220>
<223>  Primer

<400>  43
agcaacccat agtctaccct tg                                                 22

<210>  44
<211>  20
<212>  DNA
<213>  Artificial

<220>
<223>  Primer

<400>  44
cttgggctcc cctccgttat                                                    20

<210>  45
<211>  21
<212>  DNA
<213>  Artificial

<220>
<223>  Primer

<400>  45
tacccgtgga tgaccaaact g                                                  21

<210>  46
<211>  20
<212>  DNA
<213>  Artificial

<220>
<223>  Primer

<400>  46
gtctggtagc gcgtgtaact                                                    20

<210> 47
<211> 20
<212> DNA
<213> Artificial

<220>
<223> Primer

<400> 47
ttagttctga gcagggcagg 20


<210> 48
<211> 20
<212> DNA
<213> Artificial

<220>
<223> Primer

<400> 48
accgagagta gatctggcgg 20


<210> 49
<211> 20
<212> DNA
<213> Artificial

<220>
<223> Primer

<400> 49
cctccgccaa cactaacagt 20


<210> 50
<211> 20
<212> DNA
<213> Artificial

<220>
<223> Primer

<400> 50
gtaagtttgt cgaccgctgc 20


<210> 51
<211> 21
<212> DNA
<213> Artificial

<220>
<223> Primer

<400> 51
ggctccaaag tcactgcaaa t 21


<210> 52
<211> 20
<212> DNA

<213>  Artificial

<220>
<223>  Primer

<400>  52
cgtaatctgt ctctgtcggc                                                   20


<210>  53
<211>  21
<212>  DNA
<213>  Artificial

<220>
<223>  Primer

<400>  53
ccagacacct cggataacga a                                                 21


<210>  54
<211>  20
<212>  DNA
<213>  Artificial

<220>
<223>  Primer

<400>  54
tccagcgtct ggtgtttagt                                                   20


<210>  55
<211>  20
<212>  DNA
<213>  Artificial

<220>
<223>  Primer

<400>  55
aacacgaatc ccagctcgtc                                                   20


<210>  56
<211>  21
<212>  DNA
<213>  Artificial

<220>
<223>  Primer

<400>  56
aaaactctcg ctccagttcc c                                                 21


<210>  57
<211>  20
<212>  DNA
<213>  Artificial

<220>

<223>  Primer

<400>  57
gaccctggct ctctggtttc                                                   20


<210>  58
<211>  20
<212>  DNA
<213>  Artificial

<220>
<223>  Primer

<400>  58
gctgcaactt cgaatacggc                                                   20


<210>  59
<211>  20
<212>  DNA
<213>  Artificial

<220>
<223>  Primer

<400>  59
ctgggctctt tccaatggct                                                   20


<210>  60
<211>  20
<212>  DNA
<213>  Artificial

<220>
<223>  Primer

<400>  60
cttcgggctg tagaggaacc                                                   20


<210>  61
<211>  20
<212>  DNA
<213>  Artificial

<220>
<223>  Primer

<400>  61
ccacagcact ttcgagtgga                                                   20


<210>  62
<211>  20
<212>  DNA
<213>  Artificial

<220>
<223>  Primer

<400>  62

tggtgctgaa atttgtgcgg                                                    20

<210>  63
<211>  19
<212>  DNA
<213>  Artificial

<220>
<223>  Primer

<400>  63
cccaaacctg ccgaggatg                                                     19

<210>  64
<211>  22
<212>  DNA
<213>  Artificial

<220>
<223>  Primer

<400>  64
tgctgaatct tgagagagct gg                                                 22

<210>  65
<211>  20
<212>  DNA
<213>  Artificial

<220>
<223>  Primer

<400>  65
gaaggtgcac gtgaattcgg                                                    20

<210>  66
<211>  20
<212>  DNA
<213>  Artificial

<220>
<223>  Primer

<400>  66
acttgctgtc tggtgtaggc                                                    20

<210>  67
<211>  20
<212>  DNA
<213>  Artificial

<220>
<223>  Primer

<400>  67
gcccgcgaag ttttacggat                                                    20

<210> 68
<211> 20
<212> DNA
<213> Artificial

<220>
<223> Primer

<400> 68
tattgtacca gctcggcctc 20


<210> 69
<211> 20
<212> DNA
<213> Artificial

<220>
<223> Primer

<400> 69
gcagcagcaa cttgacccaa 20


<210> 70
<211> 21
<212> DNA
<213> Artificial

<220>
<223> Primer

<400> 70
ggtgtaggga cagcgctttt t 21


<210> 71
<211> 20
<212> DNA
<213> Artificial

<220>
<223> Primer

<400> 71
tgaggtttcc gtgtccagtc 20


<210> 72
<211> 20
<212> DNA
<213> Artificial

<220>
<223> Primer

<400> 72
agccaattgc tggttggagt 20


<210> 73
<211> 20
<212> DNA

<213>  Artificial

<220>
<223>  Primer

<400>  73
cccttcccat ttcccccaaa                                                   20


<210>  74
<211>  20
<212>  DNA
<213>  Artificial

<220>
<223>  Primer

<400>  74
tgccaacgcc ctaatctgtt                                                   20


<210>  75
<211>  20
<212>  DNA
<213>  Artificial

<220>
<223>  Primer

<400>  75
ggcccgctca acttgaacat                                                   20


<210>  76
<211>  20
<212>  DNA
<213>  Artificial

<220>
<223>  Primer

<400>  76
tctgctgctt tgtgtagggt                                                   20


<210>  77
<211>  20
<212>  DNA
<213>  Artificial

<220>
<223>  Primer

<400>  77
tactgtgcca aggatcagcc                                                   20


<210>  78
<211>  20
<212>  DNA
<213>  Artificial

<220>

<223>  Primer

<400>  78
gctgcagttt ggtgtaaggc                                                    20